# EUROPÄISCHE PATENTSCHRIFT

(11) **EP 2 822 537 B1**
(45) Veröffentlichungstag und Bekanntmachung des Hinweises auf die Patenterteilung: **09.12.2015**
(21) Anmeldenummer: 13727040.1
(22) Anmeldetag: 16.04.2013
(51) Int. Cl.: A61K 9/00, A61K 31/00, A61K 31/164, A61K 31/4174, A61K 31/505, A61K 31/728, A61P 11/02

(54) **KOMBINATIONSTHERAPEUTIKUM FÜR DIE BEHANDLUNG VON RHINITIS**
COMBINATION THERAPY AGENT FOR TREATING RHINITIS
AGENT DE POLYTHERAPIE POUR LE TRAITEMENT DE RHINITES

(30) Priorität: 14.05.2012 DE 102012009495; 15.05.2012 DE 102012011447
(43) Veröffentlichungstag der Anmeldung: 14.01.2015
(73) Patentinhaber: Maria Clementine Martin Klosterfrau Vertriebsgesellschaft mbH, 50670 Köln (DE)
(72) Erfinder: GREVE, Harald, 40545 Düsseldorf (DE)
(74) Vertreter: Strehlke, Ingo Kurt
(86) Internationale Anmeldenummer: PCT/EP2013/001117
(87) Internationale Veröffentlichungsnummer: WO 2013/170921

(56) Entgegenhaltungen:
- EP-A2- 0 773 022
- WO-A1-03/049747
- WO-A1-2010/015253
- DE-U1-202012 002 792
- Anonymous: "Peer Reviewed Opne Drug Database", , 1. Oktober 2004 (2004-10-01), XP002699452, Gefunden im Internet: URL:http://ch.oddb.org/de/gcc/fachinfo/reg /56839 [gefunden am 2013-06-24]

## Beschreibung

Die vorliegende Erfindung betrifft das Gebiet der Behandlung von Rhinitiden.

Die vorliegende Erfindung betrifft ein Kombinationstherapeutikum in Form einer pharmazeutischen Zusammensetzung zur Verwendung bei der topischen nasalen, bevorzugt intranasalen Behandlung von Rhinitiden zu Zwecken der Verringerung der systemischen Resorption von imidazolinbasierten alpha-Sympathomimetika.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen, allergischen oder pseudoallergischen Ursprungs sein kann. Am Häufigsten tritt eine Rhinitis im Rahmen von sogenannten Erkältungserkrankungen oder grippalen Infekten auf.

Neben einer Unterscheidung von akuter Rhinitis einerseits und chronischer Rhinitis andererseits unterscheidet man auch verschiedene Formen der Rhinitis, beispielsweise die *Rhinitis acuta, Rhinitis atrophicans, Rhinitis allergica, Rhinitis hypertrophica, Rhinitis medicamentosa, Rhinitis pseudo-membranacea, Rhinitis sicca* und *Rhinitis vasomotorica.*

Bei der sogenannten akuten Rhinitis (*Rhinitis acuta*)*,* d. h. dem gewöhnlichen Schnupfen, handelt es sich in der Regel um einen im Allgemeinen harmlosen Infekt der Nasenschleimhäute und damit um eine infektiöse Rhinitis, welche durch eine Vielzahl von Viren (insbesondere Rhinoviren und/oder Adenoviren) ausgelöst werden kann; Hauptmerkmal einer akuten Rhinitis ist eine sogenannte laufende Nase und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche im Allgemeinen mit einer Rhinitis beginnt, verschwindet aber die *Rhinitis acuta* im Allgemeinen. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter: *"Rhinitis", "Rhinitis allergica", "Rhinitis atrophicans", "Rhinitis hyperplastica", "Rhinitis pseudomemb-ranacea", "Rhinitis sicca"* und *"Rhinitis vasomotorica".*

Da es also eine Vielzahl verschiedener Typen von Viren gibt, welche eine Rhinitis auslösen können, und eine Vielzahl von Ursachen für das Auftreten einer Rhinitis existiert, können Rhinitiden, insbesondere akute Rhinitiden, im Allgemeinen nicht kausal, sondern nur symptomatisch, vorzugsweise topisch, behandelt werden, zumeist unter Anwendung von Sympathomimetika (synonym auch als sogenannte "Abschweller" oder "Dekongestiva" bezeichnet), vorzugweise alpha-Sympathomimetika, wie Xylometazolin und Oxymetazolin oder deren physiologisch verträgliche Salze.

Sympathomimetika führen aufgrund ihrer vasokonstriktorischen Eigenschaften nach lokaler bzw. topischer Anwendung in der Nase zu einer Nasenschleimhautabschwellung und einer verringerten Durchblutung der nasalen Gefäße. Üblicherweise wirken Sympathomimetika durch direkte oder indirekte Bindung an die Adrenorezeptoren, insbesondere die alpha-Adrenorezeptoren. Bei Sympathomimetika auf Basis von Imidazolinderivaten, insbesondere Xylo- bzw. Oxymetazolin, handelt es sich um direkt wirkende Sympathomimetika, welche eine Kontraktion der glatten Muskulatur bewirken. Insgesamt existieren zahlreiche Marktprodukte, welche Sympathomimetika, insbesondere Xylo- bzw. Oxymetazolin oder deren physiologisch verträgliche Salze, insbesondere deren Hydrochloride, als abschwellenden Wirkstoff zur Behandlung von Rhinitiden enthalten (beispielsweise Olynth^{®}, Nasenspray-ratiopharm^{®}, Nasivin^{®} etc.).

Problematisch am Einsatz von Sympathomimetika der vorgenannten Art ist jedoch - neben einem Austrocknen und Anschwellen der Schleimhäute - aber auch deren systemische Resorption im Körper (und dies trotz rein topischer bzw. lokaler Applikation) infolge eines Durchtritts durch die Schleimhäute. Dies kann zu gravierenden Nebenwirkungen führen, da sich die vasokonstriktorische Wirkung nicht ausschließlich auf die nasalen Gefäße beschränkt. So kann es bei übermäßiger systemischer Resorption der Sympathomimetika im Körper zu Kopfschmerzen, Schlaflosigkeit, Müdigkeit, Sehstörungen und allergischen Reaktionen kommen. Darüber hinaus kann eine übermäßige systemische Resorption von Sympathomimetika, insbesondere Xylo- bzw. Oxymetazolin, durch die infolgedessen herbeigeführten Gefäßverengungen gravierenden Einfluss auf das Herz-Kreislauf-System nehmen, da der verringerte Blutfluss kompensiert werden muss: Unter anderem kann es zu einer allgemeinen Erhöhung des Blutdrucks sowie zu einer Tachykardie kommen. Ebenfalls nachteilig ist, wenn Sympathomimetika durch systemische Ausbreitung in Akren, d. h. vom Rumpf weit entfernte und somit geringer durchblutete Körperteile, wie Finger, Zehen, Kinn und Hände, gelangen und dort eine Gefäßverengung in den bereits naturgemäß geringer durchbluteten Körperregionen bewirken.

Folglich dürfen Sympathomimetika der vorgenannten Art auch topisch nur in äußerst geringen Dosen verabreicht werden, um eine systemische Resorption in nennenswerten Mengen auszuschließen, denn - wie zuvor ausgeführt - kommt es auch bei topischer bzw. intranasaler Applikation der Sympathomimetika stets auch zu einer gewissen systemischen Resorption infolge des Durchtritts durch die Nasenschleimhäute. Infolgedessen können nicht immer die für eine optimale therapeutische Wirkung benötigten Mengen appliziert werden.

Die Internetpublikation gemäß *Open Drug Data Base* / *Medikamente* / *Fachinformationen (http:*//*ch.oddb.org*/*de*/*gcc*/*fachinfo*/*reg*/*56839)* betrifft das auf die Anmelderin selbst zurückgehende Marktprodukt "Nasic^{®}", welches zur Abschwellung der Nasenschleimhaut bei anfallsweise auftretendem Fließschnupfen (*Rhinitis vasomotorica*) und zur Behandlung der Nasenatmungsbehinderung nach operativen Eingriffen an der Nase eingesetzt werden soll. Diese Zusammensetzung basiert dabei auf Xylometazulin einerseits und Dexpanthenol andererseits.

Die WO 2010/015253 A1 betrifft eine Zusammensetzung insbesondere zur prophylaktischen bzw. kurativen Behandlung von trockener Nasenschleimhaut bzw. von Rhinitiden, wobei diese Zusammensetzung in Kombination und in jeweils pharmazeutisch wirksamen Mengen einerseits Ectoin bzw. mindestens ein Ectoinderivat sowie andererseits Pantothenol bzw. mindestens Pantothenolderivat bzw. Pantothensäure sowie deren physiologisch unbedenklichen Salze enthalten soll. Darüber hinaus kann die Zusammensetzung zudem ein Sympathomimetikum enthalten.

Weiterhin betrifft die WO 03/049747 A1 eine pharmazeutische Zusammensetzung, die Panthotenol bzw. Pantothensäure und Hyaluronsäure bzw. Hyaluronat sowie gegebenenfalls zusätzliche pharmazeutische Hilfsmittel umfasst.

Darüber hinaus betrifft die EP 0 773 022 A2, welche auf die Anmelderin selbst zurückgeht, eine pharmazeutische Zubereitung zur Behandlung akuter Rhinitiden, welche in Kombination und in physiologischer Konzentration zum einen ein zur topischen Anwendung geeignetes Sympathomimetikum mit 2-Imidazolin-Struktur sowie zum anderen Pantothenol bzw. Pantothensäure enthält.

Was schließlich die gleichermaßen auf die Anmelderin selbst zurückgehende, nachveröffentlichte und infolge der wirksamen Prioritätsbeanspruchung keinen Stand der Technik darstellende DE 20 2012 022 792 U1 anbelangt, so betrifft dieses Dokument eine pharmazeutische Zusammensetzung zur topischen nasalen Applikation für die Behandlung von Rhinitiden, wobei die Stabilität der Zusammensetzung durch die Verwendung spezieller Puffersysteme und die Einstellung eines speziellen pH-Wertes erhöht ist.

Das der vorliegenden Erfindung zugrundeliegende Problem besteht daher in der Bereitstellung einer für die topische, insbesondere nasale, bevorzugt intranasale Applikation, insbesondere für die Behandlung von Rhinitiden, geeigneten Zusammensetzung, insbesondere pharmazeutischen Zusammensetzung, welche die zuvor geschilderten Nachteile des Standes der Technik zumindest weitgehend vermeidet oder aber wenigstens abschwächt, insbesondere eine geringere systemische Resorption von Symphatomimetika, insbesondere Oxy- bzw. Xylometazolin, bei topischer bzw. lokaler Anwendung bewirkt.

Zur Lösung des zuvor geschilderten Problems schlägt die vorliegende Erfindung - gemäß einem ersten Aspekt der vorliegenden Erfindung - ein Kombinationstherapeutikum in Form einer pharmazeutischen Zusammensetzung zur Verwendung bei der topischen nasalen Behandlung von Rhinitiden zu Zwecken der Verringerung der systemischen Resorption imidazolinbasierter alpha-Sympathomimetika gemäß Anspruch 1 vor; weitere, insbesondere vorteilhafte Ausgestaltungen des erfindungsgemäßen Kombinationstherapeutikums sind Gegenstand der diesbezüglichen Unteransprüche.

Weiterhin betrifft die vorliegende Erfindung - gemäß einem zweiten Aspekt der vorliegenden Erfindung - Pantothenol zur Verwendung bei der prophylaktischen bzw. kurativen Behandlung von Rhinitiden gemäß dem diesbezüglichen unabhängigen Anspruch.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung ist somit - gemäß einem **ersten** Aspekt der vorliegenden Erfindung - ein Kombinationstherapeutikum in Form einer pharmazeutischen Zusammensetzung zur Verwendung bei der topischen nasalen Behandlung von Rhinitiden zu Zwecken der Verringerung der systemischen Resorption imidazolinbasierter alpha-Sympathomimetika,
wobei die pharmazeutische Zusammensetzung in Kombination und jeweils in pharmazeutisch wirksamen Mengen
(a)
   (a1) Pantothenol (Dexpanthenol) oder dessen physiologisch unbedenkliche Ester und/oder
   (a2) Pantothensäure oder deren physiologisch unbedenkliche Salze; und
(b) mindestens ein imidazolinbasiertes alpha-Sympathomimetikum oder dessen physiologisch unbedenkliches Salz;
enthält.

Mit anderen Worten ist Gegenstand der vorliegenden Erfindung die Verwendung von (a) (a1) Pantothenol (Dexpanthenol) oder dessen physiologisch unbedenklichen Estern bzw. (a2) von Pantothensäure oder deren physiologisch unbedenklichen Salzen zur Verringerung der systemischen Resorption eines imidazolinbasierten alpha-Sympathomimetikums bei der topischen nasalen, bevorzugt intranasalen Applikation eines mindestens ein imidazolinbasiertes alpha-Sympathomimetikum enthaltenden Arzneimittels bei der Behandlung von Rhinitiden bzw. bei der Herstellung eines Arzneimittels zur Behandlung von Rhinitiden, wobei das (a) (a1) Pantothenol (Dexpanthenol) oder dessen physiologisch unbedenkliche Ester und/oder die (a2) Pantothensäure oder deren physiologisch unbedenkliche Salze zusammen und/oder in Kombination mit (b) dem imidazolinbasierten alpha-Sympathomimetikum oder dessen physiologisch unbedenklichen Salzen verabreicht wird.

Erfindungsgemäß ist es also vorgesehen, dass (a) (a1) das Pantothenol (Dexpanthenol) oder dessen physiologisch unbedenkliche Ester und/oder die (a2) Pantothensäure oder deren physiologisch unbedenkliche Salze zur Verabreichung zusammen und/oder in Kombination mit (b) dem imidazolinbasierten alpha-Sympathomimetikum oder dessen physiologisch unbedenklichen Salzen hergerichtet ist.

Im Rahmen der vorliegenden Erfindung war es vollkommen überraschend, dass durch den zusätzlichen Einsatz von Pantothenol (Dexpanthenol) und/oder Pantothensäure die systemische Resorption von Sympathomimetika, insbesondere imidazolinbasierten Sympathomimetika, in den Blutkreislauf bei der topischen, insbesondere nasalen, bevorzugt intranasalen Applikation im Rahmen der Behandlung von Rhinitiden signifikant gesenkt werden kann. Darüber hinaus war es überraschend, dass das Sympathomimetikum in der topischen Wirksamkeit - d. h. in Bezug auf seine vasokonstriktorische bzw. abschwellende Wirkung an der Nasenschleimhaut - auch bei Einsatz der erfindungsgemäß vorgesehenen und verhältnismäßig großen Mengen von Pantothenol bzw. Pantothensäure jedoch nicht beeinträchtigt wird. Bislang war lediglich bekannt, dass Pantothenol bzw. Pantothensäure bei gemeinsamer nasaler bzw. intranasaler Applikation mit Sympathomimetika, insbesondere imidazolinbasierten Sympathomimetika, lediglich einer infolge der Verabreichung der Sympathomimetika auftretenden Austrocknung der Nasenschleimhäute entgegenwirkt.

Mit anderen Worten werden im Rahmen der vorliegenden Erfindung Pantothenol bzw. Pantothensäure in vollkommen neuartiger Verwendung eingesetzt, nämlich um eine unerwünschte systemische Resorption von Sympathomimetika, insbesondere imidazolinbasierten Sympathomimetika, bei deren topischer bzw. lokaler (z. B. intranasaler) Applikation in den Blutkreislauf in signifikantem Maße zu verringern, wobei eine derartige Optimierung neuartige aufeinander abgestimmte Mengenverhältnisse der eingesetzten Komponenten voraussetzt und auf einen neuen Ansatz mit neuartiger Wirkweise abstellt.

Denn, wie die Anmelderin überraschenderweise im Rahmen von klinischen Studien herausgefunden hat, führt die gemeinsame Verwendung von Pantothenol bzw. Pantothensäure zusammen mit Sympathomimetika, wie z. B. Xylometazolin und/oder Oxymetazolin bzw. deren Salzen, bei lokaler bzw. topischer (insbesondere intranasaler) Applikation der Sympathomimetika zu einer Verhinderung bzw. signifikanten Reduktion der systemischen Resorption der applizierten Sympathomimetika, welche ansonsten infolge des Durchtritts durch die Nasenschleimhäute stets zu beobachten ist; durch spezielle Abstimmung der Mengenverhältnisse kann eine systemische Resorption des topisch applizierten Sympathomimetikums also wirksam verhindert bzw. zumindest eingeschränkt werden.

Der Vorteil einer verringerten systemischen Resorption der Sympathomimetika liegt insbesondere darin, dass eine vasokonstriktorische Wirkung nicht auf das Herz-Kreislauf-System ausgeweitet wird und Nebenwirkungen, wie Hypertension, Tachykardie oder eine Minderdurchblutung peripherer Körperteile, vermieden werden.

Wie auch die von der Anmelderin durchgeführten und im Folgenden detailliert referierten Ausführungsbeispiele zeigen, sind die vorgenannten Wirkungen auf die zweckgerichtete und optimierte Verwendung von Pantothenol bzw. Pantothensäure in Kombination mit Sympathomimetika, insbesondere imidazolinbasierten Sympathomimetika, bei der nasalen, vorzugsweise intranasalen Applikation im Rahmen der Behandlung von Rhinitiden zurückzuführen. Insbesondere wird in diesem Zusammenhang bereits an dieser Stelle auf die von der Anmelderin durchgeführten Ausführungsbeispiele verwiesen, welche den vorgenannten Effekt in eindrucksvoller Weise belegen. Bevorzugte Ausführungsformen der vorliegenden Erfindung sind im Nachfolgenden zum besseren Verständnis dargelegt.

In Bezug auf die Komponente (a) wird diese üblicherweise aus Pantothenol (Dexpanthenol) oder dessen physiologisch unbedenklichen Estern ausgewählt und ist vorzugsweise Pantothenol (Dexpanthenol).

Als besonders vorteilhaft hat es sich erfindungsgemäß erwiesen, wenn die Komponente (b) bzw. das imidazolinbasierte alpha-Sympathomimetikum aus Xylometazolin oder Oxymetazolin ausgewählt ist, insbesondere in Form von deren physiologisch unbedenklichen Salzen, besonders bevorzugt in Form von deren Hydrochloridsalzen.

Gemäß einer besonders bevorzugten Ausführungsform ist die Komponente (b) bzw. das imidazonlinbasierte alpha-Sympathomimetikum Xylometazolin, insbesondere in Form von dessen physiologisch unbedenklichen Salzen, besonders bevorzugt in Form von dessen Hydrochloridsalz (Xylometazolinhydrochlorid).

Erfindungsgemäß ist also eine Kombination von Komponente (a) und Komponente (b) vorgesehen, um eine systemische Resorption der Komponente (b) bei topischer bzw. lokaler Anwendung zu verhindern bzw. zumindest zu verringern.

Im Rahmen der vorliegenden Erfindung ist es dabei bevorzugt, wenn (a) Pantothenol (Dexpanthenol) oder dessen physiologisch unbedenkliche Ester zusammen und/oder in Kombination mit (b) mindestens einem imidazolinbasierten alpha-Sympathomimetikum aus der Gruppe von Xylometazolin und/oder Oxymetazolin oder deren physiologisch unbedenklichen Salzen, besonders bevorzugt in Form von deren Hydrochloridsalzen, eingesetzt wird und/oder zur Verabreichung hergerichtet ist.

Insbesondere kann es im Rahmen der vorliegenden Erfindung vorgesehen sein, dass (a) Pantothenol (Dexpanthenol) oder dessen physiologisch unbedenkliche Ester, vorzugsweise Pantothenol (Dexpanthenol), zusammen und/oder in Kombination mit (b) Xylometazolin oder dessen physiologisch unbedenklichen Salzen, besonders bevorzugt in Form von dessen Hydrochloridsalz (Xylometazolinhydrochlorid), eingesetzt wird und/oder zur Verabreichung hergerichtet ist. Im Rahmen der vorliegenden Erfindung hat sich nämlich gezeigt, dass die resorptionsmindernde Wirkung von Pantothenol bzw. Pantothensäure in Bezug auf Sympathomimetika insbesondere bei Verwendung in Kombination mit imidazolinbasierten Sympathomimetika, vorzugsweise Xylometazolin, auftritt.

Erfindungsgemäß ist es vorgesehen, dass die Komponente (a) einerseits und die Komponente (b) andererseits in einer gemeinsamen pharmazeutischen Zusammensetzung vorliegen. Es ist erfindungsgemäß vorgesehen, dass die Komponente (a) einerseits und die Komponente (b) andererseits in einer gemeinsamen Zusammensetzung vorliegen, da dies eine verlässliche gemeinsame Applikation beider Komponenten (a) und (b) gewährleistet.

Mit anderen Worten ist es im Rahmen der vorliegenden Erfindung vorgesehen, dass die zuvor genannten Komponenten (a) und (b) in einer gemeinsamen Zusammensetzung in Form eines Kombinationspräparats vorliegen.

Im Zusammenhang mit der Applikation bzw. Verabreichung von Pantothenol bzw. Pantothensäure ist es erfindungsgemäß bevorzugt, wenn die Komponente (a), vorzugsweise in Form von Pantothenol (Dexpanthenol), mit einer Einzeldosis im Bereich von 0,1 mg bis 50 mg, insbesondere im Bereich von 0,5 mg bis 25 mg, vorzugsweise im Bereich von 1 mg bis 20 mg, bevorzugt im Bereich von 2,5 mg bis 10 mg, besonders bevorzugt im Bereich von 4 mg bis 8 mg, verabreicht wird bzw. wenn die Komponente (a), vorzugsweise in Form von Pantothenol (Dexpanthenol), zur Verabreichung mit einer Einzeldosis im Bereich von 0,1 mg bis 50 mg, insbesondere im Bereich von 0,5 mg bis 25 mg, vorzugsweise im Bereich von 1 mg bis 20 mg, bevorzugt im Bereich von 2,5 mg bis 10 mg, besonders bevorzugt im Bereich von 4 mg bis 8 mg, hergerichtet ist. In Bezug auf die zuvor genannten Einzeldosen ist es erfindungsgemäß zudem üblich, dass 2 bis 10 Einzeldosen pro Tag, insbesondere 3 bis 5 Einzeldosen pro Tag, verabreicht werden, vorzugsweise über den Tag verteilt, bzw. dass die Komponente (a), vorzugsweise in Form von Pantothenol (Dexpanthenol), zur Verabreichung in 2 bis 10 Einzeldosen pro Tag, insbesondere 3 bis 5 Einzeldosen pro Tag, hergerichtet ist, vorzugsweise über den Tag verteilt.

Was die Verabreichung des Sympathomimetikums anbelangt, so ist es im Rahmen der vorliegenden Erfindung bevorzugt, wenn die Komponente (b), vorzugsweise in Form von Xylometazolin und/oder Oxymetazolin oder deren physiologisch unbedenklichen Salzen, besonders bevorzugt in Form von Xylometazolinhydochlorid, mit einer Einzeldosis im Bereich von 0,001 mg bis 10 mg, insbesondere im Bereich von 0,01 mg bis 5 mg, vorzugsweise im Bereich von 0,05 mg bis 2 mg, bevorzugt im Bereich von 0,08 mg bis 1 mg, besonders bevorzugt im Bereich von 0,1 mg bis 0,8 mg, verabreicht wird bzw. wenn die Komponente (b), vorzugsweise in Form von Xylometazolin und/oder Oxymetazolin oder deren physiologisch unbedenklichen Salzen, besonders bevorzugt in Form von Xylometazolinhydochlorid, zur Verabreichung mit einer Einzeldosis im Bereich von 0,001 mg bis 10 mg, insbesondere im Bereich von 0,01 mg bis 5 mg, vorzugsweise im Bereich von 0,05 mg bis 2 mg, bevorzugt im Bereich von 0,08 mg bis 1 mg, besonders bevorzugt im Bereich von 0,1 mg bis 0,8 mg, hergerichtet ist. Im Zusammenhang mit den zuvor beschriebenen Einzeldosen des Sympathomimetikums ist es im Rahmen der vorliegenden Erfindung vorgesehen, dass üblicherweise 2 bis 10 Einzeldosen pro Tag, insbesondere 3 bis 5 Einzeldosen pro Tag, verabreicht werden, vorzugsweise über den Tag verteilt, bzw. dass die Komponente (b), vorzugsweise in Form von Xylometazolin und/oder Oxymetazolin oder deren physiologisch unbedenklichen Salzen, besonders bevorzugt in Form von Xylometazolinhydochlorid, zur Verabreichung in 2 bis 10 Einzeldosen pro Tag, insbesondere 3 bis 5 Einzeldosen pro Tag, hergerichtet ist, vorzugsweise über den Tag verteilt.

Für eine ausreichende Wirkung einerseits und eine verminderte bzw. verringerte systemische Resorption des Sympathomimetikums andererseits hat sich im Rahmen der vorliegenden Erfindung zudem gezeigt, dass insbesondere das Mengenverhältnis der Komponente (a) zu der Komponente (b) von Bedeutung ist. In diesem Zusammenhang ist es bevorzugt, wenn die Komponente (a) zusammen und/oder in Kombination mit der Komponente (b) in einem Gewichtsverhältnis von Komponente (a) zu Komponente (b) im Bereich von 10 : 1 bis 1.000 : 1, insbesondere 15 : 1 bis 500 : 1, vorzugsweise 20 : 1 bis 250 : 1, bevorzugt 25 : 1 bis 200 : 1, besonders bevorzugt 30 : 1 bis 175 : 1, ganz besonders bevorzugt 40 : 1 bis 150 : 1, noch mehr bevorzugt 45 : 1 bis 125 : 1, eingesetzt wird bzw. wenn die Komponente (a) zusammen und/oder in Kombination mit der Komponente (b) zur Verabreichung in einem Gewichtsverhältnis von Komponente (a) zu Komponente (b) im Bereich von 10 : 1 bis 1.000 : 1, insbesondere 15 : 1 bis 500 : 1, vorzugsweise 20 : 1 bis 250 : 1, bevorzugt 25 : 1 bis 200 : 1, besonders bevorzugt 30 : 1 bis 175 : 1, ganz besonders bevorzugt 40 : 1 bis 150 : 1, noch mehr bevorzugt 45 : 1 bis 125 : 1, hergerichtet ist.

Durch die vorgenannten Mengen und Mengenverhältnisse werden Wirkeffizienz und Resorptionsminderung in gleicher Weise gewährleistet. Dennoch ist es nicht ausgeschlossen, dass der Fachmann einzelfallbedingt oder anwendungsbezogen von den vorgenannten Wertebereichen abweichen kann, ohne dass er den Rahmen der vorliegenden Erfindung verlässt.

Die Menge an eingesetzter Komponente (a) kann im Rahmen der vorliegenden Erfindung in weiten Bereichen variieren. Um eine ausreichende Wirkung der Komponente (a) bei ihrer Verwendung zu gewährleisten, ist es erfindungsgemäß vorgesehen, dass die Komponente (a) in der pharmazeutischen Zusammensetzung inkorporiert ist bzw. in der pharmazeutischen Zusammensetzung verabreicht wird, wobei die Zusammensetzung, bezogen auf die Zusammensetzung, die Komponente (a) in einer Menge von 0,01 bis 10 Gew.-%, insbesondere 0,1 bis 9 Gew.-%, vorzugsweise 0,5 bis 8 Gew.-%, bevorzugt 1 bis 7 Gew.-%, besonders bevorzugt 2 bis 6 Gew.-%, ganz besonders bevorzugt 3 bis 6 Gew.-%, enthält.

Werden die vorgenannten Mengenbereiche deutlich überschritten, wird die Nasenschleimhaut - ohne sich hierbei auf diese Theorie beschränken zu wollen - derart von Pantothenol bzw. Pantothensäure belegt bzw. überlagert, dass keine Bindung der Sympathomimetika, insbesondere der imidazolinbasierter Sympathomimetika, an die Zielrezeptoren erfolgen kann, da die Diffusion durch die Haut hin zu den Rezeptoren nicht bzw. nur schlecht möglich ist. Werden die vorgenannten Mengen jedoch unterschritten, kann keine resorptionsmindernde Wirkung gewährleistet werden, da - ebenfalls ohne sich hierbei auf diese Theorie beschränken zu wollen - die Sympathomimetika vollkommen ungehindert in den systemischen Blutkreislauf diffundieren können. In diesem Zusammenhang wird bereits an dieser Stelle auf die erfindungsgemäßen Ausführungsbeispiele verwiesen.

Gleichermaßen kann auch die Menge an Komponente (b) in weiten Bereichen variieren. Im Rahmen der vorliegenden Erfindung ist es vorgesehen, dass die Komponente (b) in der pharmazeutischen Zusammensetzung inkorporiert ist bzw. in der pharmazeutischen Zusammensetzung verabreicht wird, wobei die Zusammensetzung, bezogen auf die Zusammensetzung, die Komponente (b) in einer Menge von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthält.

Wie bereits zuvor beschrieben, liegen Komponente (a) und Komponente (b) grundsätzlich in einer gemeinsamen Zusammensetzung vor. Erfindungsgemäß werden Komponente (a) und Komponente (b) ausgehend von einer gemeinsamen Zusammensetzung appliziert. Insbesondere können auf diese Weise Dosierungsfehler vermieden werden, wenn beide Komponenten in einer gemeinsamen Zusammensetzung vorliegen, da sichergestellt werden kann, dass Komponente (a) und Komponente (b) stets im optimalen und erfindungsgemäß optimierten Mengenverhältnis zueinander eingesetzt werden. Somit ist es im Rahmen der vorliegenden Erfindung vorgesehen, dass die Komponente (a) und die Komponente (b) in einer gemeinsamen pharmazeutischen Zusammensetzung inkorporiert sind bzw. dass die Komponente (a) und die Komponente (b) in der gemeinsamen pharmazeutischen Zusammensetzung verabreicht werden.

Zudem ist es im Rahmen der vorliegenden Erfindung bevorzugt, wenn die Komponente (a) zusammen und/oder in Kombination mit der Komponente (b) als (gemeinsame) wässrige Zusammensetzung und/oder in einer (gemeinsamen) wässrigen Zusammensetzung eingesetzt wird und/oder zur Verabreichung hergerichtet ist. Erfindungsgemäß kann es also vorgesehen sein, dass die Komponente (a) zusammen und/oder in Kombination mit der Komponente (b) wässrig eingesetzt wird und/oder zur Verabreichung hergerichtet ist, insbesondere in Form einer (gemeinsamen) wässrigen Lösung oder (gemeinsamen) wässrigen Solubilisierung.

Bevorzugterweise wird die Komponente (a) zusammen und/oder in Kombination mit der Komponente (b) als wässriges System, insbesondere als wässriges Einphasensystem, vorzugsweise als wässrige Lösung oder wässrige Solubilisierung, eingesetzt und/oder zur Verabreichung hergerichtet. In diesem Zusammenhang kann es gleichermaßen vorgesehen sein, dass die Komponente (a) zusammen und/oder in Kombination mit der Komponente (b) mit einem Exzipienten oder Träger auf Wasserbasis eingesetzt wird und/oder zur Verabreichung hergerichtet ist. Darüber hinaus kann es vorgesehen sein, dass die Komponente (a) zusammen und/oder in Kombination mit der Komponente (b) als klare, farblose wässrige Lösung eingesetzt wird und/oder zur Verabreichung hergerichtet ist.

Im Hinblick auf eine Stabilisierung der Komponenten (a) und (b) in der Lösung oder Solubilisierung, vor allem im Hinblick auf die Langzeitstabilität bzw. Lagerstabilität, hat sich die Einhaltung eines sehr engen pH-Regimes im Bereich von 5,0 bis 6,2 als besonders vorteilhaft erwiesen, insbesondere auch ohne den zusätzlichen Einsatz von Stabilisatoren oder Konservierungsmitteln. So ist es im Rahmen der vorliegenden Erfindung besonders vorteilhaft, wenn die Komponente (a) zusammen und/oder in Kombination mit der Komponente (b) in der pharmazeutischen Zusammensetzung mit einem pH-Wert im Bereich von 5,0 bis 6,2, insbesondere im Bereich von 5,0 bis 6,0, vorzugsweise im Bereich von 5,1 bis 6,0, bevorzugt im Bereich von 5,2 bis 5,9, eingesetzt wird bzw. zur Verabreichung hergerichtet ist. Wie die vorherigen Ausführungen zeigen, lässt sich somit insbesondere durch die Einhaltung eines leicht sauren pH-Werts eine besonders effiziente Stabilisierung beider eingesetzter Komponenten (a) und (b) erzielen. Insbesondere wird durch den vorgenannten leicht sauren pH-Bereich ein hydrolytischer Abbau der Komponenten (a) und (b) verhindert, was in einer besonders guten Lagerstabilität resultiert.

Erfindungsgemäß erfolgt im Rahmen der vorliegenden Erfindung die Bestimmung bzw. Messung des pH-Wertes mit dem Fachmann an sich bekannten bzw. geläufigen oder aber unter standardisierten Methoden, insbesondere bei 20 °C und Atmosphärendruck (1.013,25 mbar). Bekanntermaßen bezeichnet der sogenannte pH-Wert eine dimensionslose Maßzahl, welche nach DIN 19260: 1971-03 als der negative dekadische Logarithmus der Wasserstoffionen-Aktivität definiert wird. Im Rahmen der vorliegenden Erfindung bezieht sich die Angabe des pH-Werts insbesondere auf die Bestimmungsmethode gemäß DIN 19266: 2000-01, welche die ältere DIN 19266: 1979-08 ersetzt. Für weitergehende Einzelheiten zum Begriff des pH-Werts und seiner Messung und Bestimmung kann insbesondere verwiesen werden auf RÖMPP Lexikon Chemie, 10. Auflage, Georg-Thieme-Verlag, Stuttgart/New York, Band 4, 1998, Seiten 3230 bis 3232, Stichwort: "*pH*", sowie auf die dort referierte Literatur, wobei die gesamte vorgenannte Literaturstelle mit der dort referierten Literatur hiermit durch Bezugnahme eingeschlossen ist.

Im Zusammenhang mit der Stabilisierung der eingesetzten Komponenten (a) und (b) ist es darüber hinaus von Bedeutung, welche Art von Puffersystem eingesetzt wird. So ist es im Rahmen der vorliegenden Erfindung, wenn zur Einstellung und/oder Konstanthaltung des pH-Werts der pharmazeutischen Zusammensetzung mindestens ein chemisches Puffersystem, insbesondere Puffersalz(e), eingesetzt wird. Zum Begriff des chemischen Puffers bzw. der Puffersysteme kann insbesondere verwiesen werden auf RÖMPP Lexikon Chemie, 10. Auflage, Georg Thieme Verlag, Stuttgart/New York, Band 5, 1998, Seiten 3618/3619, Stichwort: "*Puffer*" sowie auf die dort referierte Literatur, deren diesbezüglicher Inhalt hiermit durch Bezugnahme eingeschlossen ist.

In diesem Zusammenhang kann es insbesondere vorgesehen sein, dass als chemisches Puffersystem bevorzugt ein Dihydrogenphosphat/Monohydrogen-phosphat-Puffersystem ("H₂PO₄⁻/HPO₄²⁻-Puffer(system)" bzw. "Phosphatpuffer(system)"), insbesondere ein Alkalidihydrogenphosphat/Alkalimonohydrogenphosphat-Puffersystem, eingesetzt wird.

Dabei hat es sich als besonders vorteilhaft erwiesen, wenn das Dihydrogenphosphat/Monohydrogenphosphat-Puffersystem mit einem Dihydrogenphosphat/Monohydrogenphosphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 100 : 1, eingesetzt wird bzw. wenn die pharmazeutische Zusammensetzung das Dihydrogenphosphat/Monohydrogenphosphat-Puffersystem mit einem Dihydrogenphosphat/Monohydrogenphosphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 100 : 1, enthält. Wie von der Anmelderin durchgeführte Stabilitätsuntersuchungen gezeigt haben, bewirkt ein derartiges Phosphat-Puffersystem eine zuverlässige Langzeitstabilisierung der Komponenten (a) und (b) auch über mehrere Monate und sogar Jahre.

Im Hinblick auf eine weitere Stabilisierung bzw. Verhinderung des Keimwachstums kann es erfindungsgemäß zudem vorgesehen sein, dass die Verabreichung der Komponenten (a) und (b) zusammen und/oder in Kombination mit (c) mindestens einem Konservierungsmittel und/oder Desinfektionsmittel erfolgt. Gleichermaßen kann es vorgesehen sein, dass die Komponenten (a) und (b) zur Verabreichung zusammen und/oder in Kombination mit (c) mindestens einem Konservierungsmittel und/oder Desinfektionsmittel hergerichtet sind. Im Übrigen haben die Untersuchungen der Anmelderin überraschenderweise ergeben, dass die zusätzliche Anwesenheit eines Konservierungsmittels bzw. Desinfektionsmittels, insbesondere auf Basis von Benzalkoniumchlorid, eine weitergehende Reduktion der systemischen Resorption des lokal bzw. topisch verabreichten Sympathomimetikums bewirkt; ein derartiger Effekt des zusätzlich eingesetzten Konservierungsmittels bzw. Desinfektionsmittels war vollkommen überraschend und nicht vorhersehbar.

In diesem Zusammenhang ist es üblicherweise vorgesehen, dass die Komponenten (a), (b) und (c) in der pharmazeutischen Zusammensetzung inkorporiert sind bzw. vorliegen bzw. in der gemeinsamen pharmazeutischen Zusammensetzung verabreicht werden.

Was den Einsatz eines Konservierungsmittels bzw. Desinfektionsmittels als Komponente (c) im Speziellen anbelangt, so ist es im Rahmen der vorliegenden Erfindung bevorzugt, wenn das Konservierungsmittel bzw. Desinfektionsmittel aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) para-Hydroxybenzoesäureestern und Mischungen von verschiedenen para-Hydroxybenzoesäureestern, vorzugsweise Nipa-Estern, (iii) Chlorhexidin sowie Kombinationen der vorgenannten Verbindungen und besonders bevorzugt Benzalkoniumchlorid ausgewählt ist. Denn im Rahmen der vorliegenden Erfindung hat die Anmelderin überraschenderweise herausgefunden, dass die zusätzliche Verwendung von Konservierungs- bzw. Desinfektionsmitteln, insbesondere Benzalkoniumchlorid, die Komponenten (a) und (b) in ihrer Wirkung weiter verstärkt. Insbesondere ist die Verwendung von Konservierungs- bzw. Desinfektionsmitteln mit dem Vorteil verbunden, dass aufgrund des desinfizierenden Effekts die Wirkung der Komponenten (a) und (b) unterstützt wird und darüber hinaus einem Keimwachstum - sowohl bei Lagerung als auch nach Applikation auf die Schleimhäute - in effizienter Weise entgegengewirkt wird.

Die eingesetzte Menge von Komponente (c), insbesondere Benzalkoniumchlorid, ist gleichermaßen variabel. Insbesondere kann es vorgesehen sein, dass die Komponente (c), vorzugsweise in Form von Benzalkoniumchlorid, mit einer Einzeldosis im Bereich von 0,001 mg bis 5 mg, insbesondere im Bereich von 0,005 mg bis 2 mg, vorzugsweise im Bereich von 0,01 mg bis 1 mg, verabreicht wird bzw. wenn die Komponente (c), vorzugsweise in Form von Benzalkoniumchlorid, zur Verabreichung mit einer Einzeldosis im Bereich von 0,005 mg bis 2 mg, vorzugsweise im Bereich von 0,01 mg bis 1 mg, hergerichtet ist; dabei können insbesondere 2 bis 10 Einzeldosen pro Tag, insbesondere 3 bis 5 Einzeldosen pro Tag, verabreicht werden, vorzugsweise über den Tag verteilt. Mit anderen Worten kann es dabei insbesondere vorgesehen sein, dass die Komponente (c), vorzugsweise in Form von Benzalkoniumchlorid, zur Verabreichung in 2 bis 10 Einzeldosen pro Tag, insbesondere 3 bis 5 Einzeldosen pro Tag, hergerichtet ist, vorzugsweise über den Tag verteilt.

Darüber hinaus ist es im Rahmen der vorliegenden Erfindung üblich, dass die Komponente (c) in der pharmazeutischen Zusammensetzung inkorporiert ist bzw. dass die Komponente (c) in der pharmazeutischen Zusammensetzung verabreicht wird, wobei die Zusammensetzung, bezogen auf die Zusammensetzung, die Komponente (c) in einer Menge von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, enthält. In diesem Fall enthält die Zusammensetzung also bevorzugt die Komponenten (a), (b) und (c).

Im Rahmen der vorliegenden Erfindung ist es somit insbesondere von Vorteil, wenn die Komponenten (a), (b) und (c) in der gemeinsamen pharmazeutischen Zusammensetzung inkorporiert sind bzw. vorliegen und/oder in der gemeinsamen pharmazeutischen Zusammensetzung verabreicht werden.

Wie die Anmelderin gleichermaßen überraschenderweise herausgefunden hat, können auch Glykosaminoglykane, insbesondere Hyaluronsäure bzw. deren Salze, die Wirkung der übrigen Komponenten, insbesondere des Pantothenols bzw. der Pantothensäure, unterstützen. Darüber hinaus können die vorgenannten Glykosaminoglykane, vorzugsweise die Hyaluronsäure, förderlich in Bezug auf die Linderung von Infektionen und Entzündungen der Nasenschleimhaut wirken - vermutlich aufgrund ihrer Fähigkeit, große Mengen Flüssigkeit zu binden - und in der Folge eine Beschleunigung der Regeneration von entzündetem Gewebe bewirken. Zudem bewirken die Glykosaminoglykane, insbesondere Hyaluronsäure bzw. deren Salze, zusammen mit der Komponente (a) eine weitergehende Verringerung bzw. Reduktion einer unerwünschten systemischen Resorption der topisch bzw. lokal applizierten Sympathomimetika.

Im Rahmen der vorliegenden Erfindung hat es sich in diesem Zusammenhang als vorteilhaft erwiesen, wenn die Verabreichung der Komponenten (a) und (b) und gegebenenfalls (c) zusammen und/oder in Kombination mit (d) mindestens einem vorzugsweise sauren Glykosaminoglykan oder dessen physiologisch unbedenklichen Salzen oder Derivaten, insbesondere Hyaluronsäure oder deren physiologisch unbedenklicher Salze, erfolgt bzw. dass die Komponenten (a) und (b) und gegebenenfalls (c) zur Verabreichung zusammen und/oder in Kombination mit (d) mindestens einem vorzugsweise sauren Glykosaminoglykan oder dessen physiologisch unbedenklichen Salzen oder Derivaten, insbesondere Hyaluronsäure oder deren physiologisch unbedenklichen Salzen, hergerichtet sind. Dabei ist es insbesondere von Vorteil, wenn die Komponenten (a), (b), (c) und (d) in der gemeinsamen pharmazeutischen Zusammensetzung inkorporiert sind bzw. vorliegen und/oder in der gemeinsamen pharmazeutischen Zusammensetzung verabreicht werden.

Was die eingesetzten Mengen der vorgenannten Glykosaminoglykane anbelangt, so können auch diese in weiten Bereichen variieren. Insbesondere kann es im Rahmen der vorliegenden Erfindung vorgesehen sein, dass die Komponente (d) in der pharmazeutischen Zusammensetzung inkorporiert ist und/oder die Komponente (d) in der pharmazeutischen Zusammensetzung verabreicht wird, wobei die Zusammensetzung, bezogen auf die Zusammensetzung, die Komponente (d) in einer Menge von 0,0001 bis 10 Gew.-%, insbesondere 0,001 bis 5 Gew.-%, vorzugsweise 0,01 bis 2 Gew.-%, enthält.

Im Rahmen der vorliegenden Erfindung kann es weiterhin vorgesehen sein, dass die Verabreichung der Komponenten (a) und (b) und gegebenenfalls (c) und/oder (d) zusammen und/oder in Kombination mit (e) Ectoin oder mindestens einem Ectoinderivat, insbesondere einem Hydroxyectoin, erfolgt bzw. dass die Komponenten (a) und (b) und gegebenenfalls (c) und/oder (d) zur Verabreichung zusammen und/oder in Kombination mit (e) Ectoin oder mindestens einem Ectoinderivat, insbesondere einem Hydroxyectoin, hergerichtet sind. In diesem Zusammenhang kann es insbesondere vorgesehen sein, dass die Komponenten (a) und (b) sowie gegebenenfalls (c) und/oder gegebenenfalls (d) und die Komponente (e) in der gemeinsamen pharmazeutischen Zusammensetzung inkorporiert sind bzw. vorliegen und/oder in einer gemeinsamen pharmazeutischen Zusammensetzung verabreicht werden.

Denn, wie die Anmelderin überraschend gefunden hat, ist die Verwendung von Ectoin bzw. von Ectoinderivaten im Hinblick auf eine Beschleunigung der Regeneration von entzündeter und/oder wunder Nasenschleimhaut, wie sie häufig im Rahmen von Rhinitiden auftritt, von Vorteil. Auch unterstützen Ectoin bzw. Ectoinderivate die Verminderung der systemischen Resorption lokal applizierter Sympathomimetika.

Die eingesetzte Menge an Ectoin ist in weiten Bereichen variabel. Üblicherweise ist es im Rahmen der vorliegenden Erfindung vorgesehen, dass die Komponente (e) in der pharmazeutischen Zusammensetzung inkorporiert ist und/oder die Komponente (e) in der pharmazeutischen Zusammensetzung verabreicht wird, wobei die Zusammensetzung, bezogen auf die Zusammensetzung, die Komponente (e) in einer Menge von 0,0001 bis 10 Gew.-%, insbesondere 0,001 bis 5 Gew.-%, vorzugsweise 0,01 bis 2 Gew.-%, enthält.

Darüber hinaus kann es im Rahmen der vorliegenden Erfindung vorgesehen sein, dass die Verabreichung der Komponenten (a) und (b) und gegebenenfalls (c), (d) und/oder (e) zusammen und/oder in Kombination mit (f) Natriumchlorid erfolgt bzw. dass die Komponenten (a) und (b) und gegebenenfalls (c), (d) und/oder (e) zur Verabreichung zusammen und/oder in Kombination mit (f) Natriumchlorid hergerichtet sind. Auch in diesem Zusammenhang ist es üblich, dass die Komponenten (a) und (b) sowie gegebenenfalls (c), (d) und/oder (e) und die Komponente (f) in der gemeinsamen pharmazeutischen Zusammensetzung inkorporiert sind bzw. vorliegen und/oder in der gemeinsamen pharmazeutischen Zusammensetzung verabreicht werden. Natriumchlorid bewirkt insbesondere eine Befeuchtung der Nasenschleimhäute und trägt somit ebenfalls zu einer beschleunigten Regeneration von entzündeter Nasenschleimhaut bei.

Was die Menge an eingesetztem Natriumchlorid anbelangt, so kann diese in weiten Bereichen variieren. Insbesondere kann es erfindungsgemäß vorgesehen sein, dass die Komponente (f) in der pharmazeutischen Zusammensetzung inkorporiert ist und/oder dass die Komponente (f) in der pharmazeutischen Zusammensetzung verabreicht wird, wobei die Zusammensetzung, bezogen auf die Zusammensetzung, die Komponente (e) in einer Menge von 0,001 bis 5 Gew.-%, insbesondere 0,01 bis 2 Gew.-%, vorzugsweise 0,1 bis 1 Gew.-%, enthält.

Weiterhin kann es erfindungsgemäß vorgesehen sein, dass die Komponente (a) zusammen und/oder in Kombination mit der Komponente (b) mit mindestens einem weiteren Inhaltsstoff eingesetzt wird bzw. zur Verabreichung hergerichtet ist. In diesem Zusammenhang ist es besonders bevorzugt, wenn der weitere Inhaltsstoff aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen ausgewählt wird.

Darüber hinaus hat sich im Rahmen der vorliegenden Erfindung gezeigt, dass durch Einstellung einer Osmolalität in definierten Bereichen eine gute Verträglichkeit auf den Nasenschleimhäuten gewährleistet wird. Die Osmolalität kann - wie die folgenden Ausführungen zeigen - in weiten Bereich variieren. Erfindungsgemäß hat es sich als vorteilhaft erwiesen, wenn alle Wirk- und/oder Inhaltsstoffe, insbesondere die Komponenten (a) und (b) und gegebenenfalls (c), (d), (e) und/oder (f), in der gemeinsamen pharmazeutischen Zusammensetzung inkorporiert sind und/oder verabreicht werden, wobei die Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist.

Im Rahmen der vorliegenden Erfindung ist es zudem bevorzugt, wenn alle Wirk- und/oder Inhaltsstoffe, insbesondere die Komponenten (a) und (b) und gegebenenfalls (c), (d), (e) und/oder (f), in der gemeinsamen pharmazeutischen Zusammensetzung inkorporiert sind und/oder verabreicht werden, wobei die Zusammensetzung bei einer Temperatur von 20 °C und bei einem Druck von 1.013,25 mbar eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist.

Darüber hinaus sind die eingesetzten Komponenten, insbesondere die Komponenten (a) und (b), im Rahmen der vorliegenden Erfindung unter Normalbedingungen über einen äußerst langen Zeitraum stabil, insbesondere lagerstabil. So ist es erfindungsgemäß üblich, dass alle Wirk- und/oder Inhaltsstoffe, insbesondere die Komponenten (a) und (b) und gegebenenfalls (c), (d), (e) und/oder (f), in der gemeinsamen pharmazeutischen Zusammensetzung inkorporiert sind bzw. verabreicht werden, wobei die Zusammensetzung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil, ist.

Wie die vorstehenden Ausführungen zeigen, zeichnen sich im Rahmen der vorliegenden Erfindung somit alle Wirk- bzw. Inhaltsstoffe, insbesondere das Pantothenol bzw. die Pantothensäure sowie das imidazolinbasierte Sympathomimetikum, durch eine hervorragende Stabilität bzw. durch einen äußerst geringen Gehalt an Abbauprodukten aus.

Insbesondere entstehen auch bei langfristiger Lagerung nur in äußerst geringfügigem Maße Abbauprodukte der Komponenten (a) bzw. (b):
Imidazolinbasierte Sympathomimetika, wie Xylo- oder Oxymetazolin, werden im Allgemeinen durch hydrolytische Spaltung des Imidazolringes unter Bildung des entsprechenden Amids abgebaut. Im Folgenden werden derartige Abbauprodukte als "Verunreinigung A" bezeichnet. So ist es im Rahmen der vorliegenden Erfindung üblich, dass alle Wirk- und/oder Inhaltsstoffe, insbesondere die Komponenten (a) und (b) und gegebenenfalls (c), (d), (e) und/oder (f), in der gemeinsamen pharmazeutischen Zusammensetzung inkorporiert sind und/oder verabreicht werden, wobei die Zusammensetzung einen Gehalt an Abbauprodukt(en) der Komponente (b), insbesondere Verunreinigung A, von höchstens 5 Gew.-%, insbesondere von höchstens 3 Gew.-%, vorzugsweise von höchstens 2 Gew.-%, besonders bevorzugt von höchstens 1 Gew.-%, bezogen auf den Wirkstoff (b), aufweist, insbesondere auch nach Lagerung des Arzneimittels bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten.

Bei Abbau der Komponente (a) bzw. Pantothenol oder Pantothensäure hingegen entsteht als Abbauprodukt Aminopropanol und/oder D-Pantolacton. Üblicherweise verhält es sich im Rahmen der vorliegenden Erfindung derart, dass alle Wirk- und/oder Inhaltsstoffe, insbesondere die Komponenten (a) und (b) und gegebenenfalls (c), (d), (e) und/oder (f), in der gemeinsamen pharmazeutischen Zusammensetzung inkorporiert sind und/oder verabreicht werden, wobei die Zusammensetzung einen Gehalt an Abbauprodukt(en) der Komponente (a), insbesondere Aminopropanol und/oder D-Pantolacton, von jeweils höchstens 5 Gew.-%, insbesondere von jeweils höchstens 3 Gew.-%, vorzugsweise von jeweils höchstens 2 Gew.-%, besonders bevorzugt von jeweils höchstens 1 Gew.-%, bezogen auf den Wirkstoff (a), aufweist, insbesondere auch nach Lagerung des Arzneimittels bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten. Auch die Komponente (a) zeichnet sich somit durch eine hervorragende Stabilität aus.

Im Rahmen der vorliegenden Erfindung ist insbesondere die Verwendung der zuvor beschriebenen Zusammensetzung zur Behandlung von Rhinitiden aller Art, insbesondere *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta* vorgesehen. Insbesondere ist das erfindungsgemäße Kombinationstherapeutikum jedoch für eine prophylaktische und/oder kurative topische Behandlung von Rhinitiden, insbesondere *Rhinitis acuta,* geeignet.

Die Verabreichung der im Rahmen der vorliegenden Erfindung beschriebenen Zusammensetzungen erfolgt mit für die topische, insbesondere nasale, Applikation üblichen Vorrichtungen. So kann im Rahmen der vorliegenden Erfindung vorgesehen sein, dass alle Wirk- und/oder Inhaltsstoffe, insbesondere die Komponenten (a) und (b) und gegebenenfalls (c), (d), (e) und/oder (f), in der gemeinsamen pharmazeutischen Zusammensetzung inkorporiert sind und/oder verabreicht werden, wobei die Zusammensetzung mittels einer Applikationsvorrichtung, bevorzugt in Form eines Behältnisses mit Tropf- oder Sprüheinrichtung, verabreicht wird und/oder zur Verabreichung hergerichtet ist.

Um eine ausreichende Menge zu applizieren, ist es erfindungsgemäß bevorzugt, wenn die Applikationsvorrichtung eine Sprüheinrichtung zur gleichförmigen Ausbringung der Zusammensetzung in einer Menge pro Sprühstoß im Bereich von 25 µl bis 300 µl, insbesondere im Bereich von 50 µl bis 200 µl, vorzugsweise im Bereich von 75 µl bis 125 µl, aufweist.

Gleichermaßen ist es in diesem Zusammenhang üblicherweise vorgesehen, dass die Applikationsvorrichtung einen Vorratsbehälter mit einem Volumen im Bereich von 5 ml bis 100 ml, insbesondere 10 ml bis 50 ml, aufweist.

Wie die vorstehenden Ausführungen zeigen, ist es im Rahmen der vorliegenden Erfindung erstmalig gelungen, ein vollkommen neuartiges Konzept des Einsatzes von Pantothenol bzw. Pantothensäure im Zusammenhang mit einer Verringerung der systemischen Resorption von imidazolinbasierten Sympathomimetika, insbesondere Xylometazolin, bereitzustellen. Dieses neuartige Konzept erlaubt die Applikation ausreichender Mengen des Sympathomimetikums zur Erzielung einer hervorragenden vasokonstriktorischen Wirkung bei gleichzeitiger Senkung des Auftretens von Nebenwirkungen, insbesondere Nebenwirkungen des Herz-Kreislaufsystems, bei der nasalen, insbesondere intranasalen Applikation zur Behandlung von Rhinitiden aller Art, durch Reduzierung der systemischen Resorption des Sympathomimetikums.

Für weitergehende Einzelheiten zu dem erfindungsgemäßen Kombinationstherapeutikum kann auf die nachfolgenden Ausführungen zu dem weiteren Erfindungsaspekt verwiesen werden, welche in Bezug auf das erfindungsgemäße Kombinationstherapeutikum entsprechend gelten.

Des Weiteren betrifft die vorliegende Erfindung - gemäß einem **zweiten** Aspekt der vorliegenden Erfindung - Pantothenol (Dexpanthenol) oder dessen physiologisch unbedenkliche Ester und/oder Pantothensäure oder deren physiologisch unbedenkliche Salze zur Verwendung bei der prophylaktischen und/oder kurativen Behandlung von Rhinitiden mittels nasaler, bevorzugt intranasaler Applikation zu Zwecken der Verringerung der systemischen Resorption eines imidazolinbasierten alpha-Sympathomimetikums, wobei das Pantothenol (Dexpanthenol) oder dessen physiologisch unbedenkliche Ester und/oder die Pantothensäure oder deren physiologisch unbedenkliche Salze zusammen und/oder in Kombination mit dem imidazolinbasierten alpha-Sympathomimetikum oder dessen physiologisch unbedenklichen Salzen verabreicht wird.

Gleichermaßen betrifft die vorliegende Erfindung Pantothenol (Dexpanthenol) oder dessen physiologisch unbedenkliche Ester und/oder Pantothensäure oder deren physiologisch unbedenkliche Salze zur Verwendung bei der nasalen, bevorzugt intranasalen Behandlung von Rhinitiden zusammen und/oder in Kombination mit mindestens einem imidazolinbasierten alpha-Sympathomimetikum oder dessen physiologisch unbedenklichen Salzen zu Zwecken der Verringerung der systemischen Resorption des imidazolinbasierten alpha-Sympathomimetikums.

Für weitergehende Einzelheiten zu dem zweiten Aspekt der vorliegenden Erfindung kann auf die vorangehenden Ausführungen zu dem erfindungsgemäßen Kombinationstherapeutikum verwiesen werden, welche in Bezug auf den zweiten Erfindungsaspekt entsprechend gelten.

Vorliegend wird zudem ein Verfahren zur Behandlung von Rhinitiden mittels nasaler, bevorzugt intranasaler Applikation eines mindestens ein imidazolinbasiertes alpha-Sympathomimetikum oder dessen physiologisch unbedenkliche Salze enthaltenden Arzneimittels, insbesondere Rhinologikums, beschrieben, wobei zur Verringerung der systemischen Resorption des imidazolinbasierten alpha-Sympathomimetikums zusammen und/oder in Kombination mit dem imidazolinbasierten alpha-Sympathomimetikum mindestens eine Verbindung aus der Gruppe von Pantothenol (Dexpanthenol) oder dessen physiologisch unbedenklichen Estern und/oder Pantothensäure oder deren physiologisch unbedenklichen Salzen verabreicht wird.

Für weitergehende Einzelheiten zu dem Verfahren kann auf die vorangehenden Ausführungen zu den übrigen Erfindungsaspekten verwiesen werden, welche in Bezug auf das Verfahren entsprechend gelten.

Im Rahmen der vorliegenden Erfindung ist somit erstmalig ein effizientes Therapiekonzept bzw. Kombinationstherapeutikum zur Verringerung einer systemischen Resorption von imidazolinbasierten Sympathomimetika bei deren topischer Anwendung bereitgestellt worden. Wie die Anmelderin überraschend herausgefunden hat, bewirkt nämlich die kombinierte Gabe von Pantothenol (Dexpanthenol) bzw. Pantothensäure mit Sympathomimetika eine signifikante Reduktion, wenn nicht sogar Verhinderung einer systemischen Resorption des lokal verabreichten Sympathomimetikums. Dies war vollkommen überraschend und in dieser Weise nicht vorhersehbar. Durch optimierte Einstellung der Wirkstoffmengen an den beiden vorgenannten Komponenten (a) und (b) kann somit erstmals ein wirkoptimiertes Kombinationstherapeutikum bereitgestellt werden, welches eine optimale Gabe von Sympathomimetika im Rahmen der Behandlung von Rhinitiden erlaubt.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### Ausführungsbeispiele:

### 1. Herstellungsbeispiele:

### Allgemeine Herstellungsvorschrift

Zur Herstellung von jeweils 1.000 g einer klaren wässrigen Lösung der Zusammensetzung gemäß der vorliegenden Erfindung wird in dem Fachmann an sich bekannter Weise vorgegangen: Zunächst wird bei Raumtemperatur und Umgebungsdruck in einem entsprechenden Reaktionsgefäß bzw. Glasgefäß mit Rühreinrichtung eine definierte Menge an gereinigtem Wasser (z. B. 300 ml bis 600 ml) vorgelegt und nachfolgend mit einer Lösung eines Puffersystems auf Basis von Kaliumdihydrogenphosphat/Natriummonohyrogenphospat (Dodekahydrat) auf einen vorgegebenen pH-Wert unter anschließender Kontrolle des erreichten pH-Werts eingestellt. Die eingestellten pH-Werte variieren in den unter den Rezepturbeispielen angegebenen Wertebereichen. Anschließend wird hierzu eine wie nachfolgend in den Rezepturbeispielen spezifizierte Dexpanthenolmenge hinzugegeben und unter gründlichem Rühren klar gelöst. Der Lösung wird dann gegebenenfalls ein Konservierungs- bzw. Desinfektionsmittel, vorzugsweise Benzalkoniumchlorid, bevorzugt in Form einer wässrigen Lösung, zugesetzt und gleichfalls unter Rühren gelöst. Nachfolgend wird die gewünschte Xylometazolinhydrochloridmenge zugesetzt, mit weiterem Wasser zum Endgewicht von 1.000 g aufgefüllt und homogen gerührt. Gegebenenfalls wird die Lösung über neutrale Cellulosefilter filtriert. Abschließend wird der pH-Wert noch einmal kontrolliert. Auch die übrigen Spezifikationen der Lösung werden auf die eingestellten bzw. vorgewählten Wertebereiche hin überprüft (z. B. Osmolalität: 350 bis 395 mosm/kg; relative Dichte bei 20 °C: 1,01 bis 1,05; mikrobiologische Reinheit und Sterilität; Ausschluss von Verunreinigungen, insbesondere Abbauprodukten der eingesetzten Komponenten). Ein Teil der erhaltenen Lösung wird schließlich in Enghalsflaschen aus Braunglas zu 10 ml oder 20 ml abgefüllt, welche wahlweise mit einer Tropfpipette oder einer Sprühdosierpumpe ausgerüstet werden können; zur bestimmungsgemäßen Anwendung können mehrmals täglich 1 bis 3 Tropfen bzw. 1 bis 3 Sprühstöße à 75 bis 125 µl in jedes Nasenloch gegeben und aufgezogen werden.

Nach dieser allgemeinen Herstellungsvorschrift werden die nachfolgend spezifizierten Rezepturen zur Untersuchung des Einflusses von (a) der eingesetzten Menge an Dexpanthenol, (b) des Einflusses des pH-Wertes und (c) des Einflusses von Konservierungsmitteln hergestellt.

### a) Einfluss der eingesetzten Mengen an Dexpanthenol

Um den Einfluss der eingesetzten Menge an Dexpanthenol zu untersuchen, werden sechs Zusammensetzungen gemäß der zuvor geschilderten Herstellungsvorschrift bereitgestellt, welche jeweils 0 Gew.-% (nicht erfindungsgemäß, Rezeptur A), 0,1 Gew.-% (Rezeptur B), 3,0 Gew.-% (Rezeptur C), 5,0 Gew.-% (Rezeptur D), 6,0 Gew.-% (Rezeptur E) sowie 9,0 Gew.-% (Rezeptur F) Dexpanthenol enthalten. Darüber hinaus enthalten die Zusammensetzungen jeweils 0,1 Gew.-% Xylometazolinhydrochlorid und werden jeweils auf einen pH-Wert im Bereich von 5,2 bis 5,9, jeweils eine Osmolalität im Bereich 350 bis 395 mosm/kg sowie jeweils eine relative Dichte bei 20 °C im Bereich von 1,005 bis 1,105 eingestellt. Die genauen Angaben in Bezug auf die vorgenannten Zusammensetzungen lassen sich den nachfolgenden Tabellen entnehmen. Die angegebenen Mengenangaben beziehen sich jeweils auf 10 g Zusammensetzung.

Nichterfindungsgemäße Rezeptur A (ohne Dexpanthenol) zum Vergleich:

| **Inhaltsstoff** | **Menge/mg** | **Qualität** |
|---|---|---|
| Xylometazolinhydrochlorid | 10,00 | Ph. Eur. |
| Dexpanthenol | 0 | Ph. Eur. |
| Puffersystem | 89,30 | Ph. Eur. |
| Kaliumdihydrogenphosphat/ Natriummonohydrogenphosphat (Dodekahydrat) | 2,70 | |
| gereinigtes Wasser | 9.902 | Ph. Eur. |

### Erfindungsgemäße Rezepturen:

### Rezeptur B (0,1 Gew.-% Dexpanthenol)

| **Inhaltsstoff** | **Menge/mg** | **Qualität** |
|---|---|---|
| Xylometazolinhydrochlorid | 10,00 | Ph. Eur. |
| Dexpanthenol | 10 | Ph. Eur. |
| Puffersystem | 89,30 | Ph. Eur. |
| Kaliumdihydrogenphosphat/ Natriummonohydrogenphosphat (Dodekahydrat) | 2,70 | |
| gereinigtes Wasser | 9.892 | Ph. Eur. |

### Rezeptur C (3,0 Gew.-% Dexpanthenol)

| **Inhaltsstoff** | **Menge/mg** | **Qualität** |
|---|---|---|
| Xylometazolinhydrochlorid | 10,00 | Ph. Eur. |
| Dexpanthenol | 300 | Ph. Eur. |
| Puffersystem | 89,30 | Ph. Eur. |
| Kaliumdihydrogenphosphat/ Natriummonohydrogenphosphat (Dodekahydrat) | 2,70 | |
| gereinigtes Wasser | 9.602 | Ph. Eur. |

### Rezeptur D (5,0 Gew.-% Dexpanthenol)

| **Inhaltsstoff** | **Menge/mg** | **Qualität** |
|---|---|---|
| Xylometazolinhydrochlorid | 10,00 | Ph. Eur. |
| Dexpanthenol | 500 | Ph. Eur. |
| Puffersystem | 89,30 | Ph. Eur. |
| Kaliumdihydrogenphosphat/ Natriummonohydrogenphosphat (Dodekahydrat) | 2,70 | |
| gereinigtes Wasser | 9.402 | Ph. Eur. |

### Rezeptur E (6,0 Gew-% Dexpanthenol)

| **Inhaltsstoff** | **Menge/mg** | **Qualität** |
|---|---|---|
| Xylometazolinhydrochlorid | 10,00 | Ph. Eur. |
| Dexpanthenol | 600 | Ph. Eur. |
| Puffersystem | 89,30 | Ph. Eur. |
| Kaliumdihydrogenphosphat/ Natriummonohydrogenphosphat (Dodekahydrat) | 2,70 | |
| gereinigtes Wasser | 9.302 | Ph. Eur. |

### Rezeptur F (9,0 Gew.-% Dexpanthenol)

| **Inhaltsstoff** | **Menge/mg** | **Qualität** |
|---|---|---|
| Xylometazolinhydrochlorid | 10,00 | Ph. Eur. |
| Dexpanthenol | 900 | Ph. Eur. |
| Puffersystem | 89,30 | Ph. Eur. |
| Kaliumdihydrogenphosphat/ Natriummonohydrogenphosphat (Dodekahydrat) | 2,70 | |
| gereinigtes Wasser | 9.002 | Ph. Eur. |

### b) Einfluss des pH-Wertes

Um den Einfluss des pH-Wertes auf die Wirksamkeit der Zusammensetzung zu untersuchen, werden zwei weitere Zusammensetzungen (Rezepturen G und H), enthaltend eine Wirkstoffkombination auf Basis von 5 Gew.-% Dexpanthenol und 0,1 Gew.-% Xylometazolinhydrochlorid, mit einer Osmolalität im Bereich von 350 bis 395 mosm/kg sowie einer Dichte im Bereich von 1,005 bis 1,105, aufweisend einen pH-Wert von 5,0 (Rezeptur G) bzw. 6,2 (Rezeptur H), hergestellt. Es wird ebenfalls ein Puffersystem auf Basis von Kaliumdihydrogenphosphat/Natriummonohydrogenphosphat (Dodekahydrat) eingesetzt. Abgesehen von den pH-Werten entsprechen die Zusammensetzungen hinsichtlich ihrer Inhaltsstoffe somit der oben bereits beschriebenen Zusammensetzung mit einer Dexpanthenolmenge von 5,0 Gew.-% (Rezeptur D), auf welche zur Vermeidung unnötiger Wiederholungen verwiesen wird.

### c) Einfluss des Einsatzes von Benzalkoniumchlorid

Um den Einfluss von Konservierungs- bzw. Desinfektionsmitteln auf Basis von Benzalkoniumchlorid zu untersuchen, wird eine Zusammensetzung hergestellt, welche 5,0 Gew.-% Dexpanthenol, 0,1 Gew.-% Xylometazolin sowie 0,02 Gew.-% Benzalkoniumchlorid als 50 %-ige Lösung enthält (Rezeptur I). Der pH-Wert der Zusammensetzung liegt im Bereich von 5,2 bis 5,9, die Dichte im Bereich von 1,005 bis 1,105 und die Osmolalität im Bereich von 350 bis 395 mosm/kg. Die Rezeptur der Zusammensetzung ist der nachfolgenden Tabelle zu entnehmen.

### Rezeptur I (0,02 Gew.-% Benzalkoniumchlorid)

| **Inhaltsstoff** | **Menge/mg** | **Qualität** |
|---|---|---|
| Xylometazolinhydrochlorid | 10,00 | Ph. Eur. |
| Dexpanthenol | 500 | Ph. Eur. |
| Puffersystem | 89,30 | Ph. Eur. |
| Kaliumdihydrogenphosphat/ Natriummonohydrogenphosphat (Dodekahydrat) | 2,70 | |
| Konservierungs-/Desinfektionsmittel: Benzalkoniumchlorid (als 50 %-ige Lösung) | 4,00 | Ph. Eur. |
| gereinigtes Wasser | 9.398 | Ph. Eur. |

### 2. Anwendungsbeobachtungen:

Um die Wirksamkeit der Testzusammensetzungen zu kontrollieren, wurden je 12 Probanden eines aus 108 Patienten bestehenden Probandenkollektivs im Alter von 20 bis 73 Jahren (66 männlich, 42 weiblich), welche an akuten Rhinitiden litten, mit jeweils einer der im vorliegenden Abschnitt 1.) beschriebenen Rezepturen A bis I für die Dauer der Erkrankung (im Allgemeinen 3 bis 7 Tage) behandelt; die Rezepturen wurden mehrmals täglich als Spray topisch appliziert. Mit allen eingesetzten Zusammensetzungen konnte im Allgemeinen eine gute vasokonstriktorische Wirkung, d. h. eine zufriedenstellende Abschwellung der Nasenschleimhäute, erzielt werden.

Besonders gute Ergebnisse hinsichtlich der abschwellenden Wirkung einerseits sowie dem Verhindern der Austrocknung der Nasenschleimhaut andererseits wurden mit den erfindungsgemäßen Zusammensetzungen B, D, E, G und H erzielt.

Bei den Probanden, welche die Zusammensetzung gemäß der nichterfindungsgemäßen Rezeptur A getestet hatten, war aufgrund der fehlenden Wirkung von Dexpanthenol erfindungsgemäß ein stärkeres Austrocknen der Nasenschleimhaut zu beobachten.

Bei den Probanden, welche die Zusammensetzung gemäß der erfindungsgemäßen Rezeptur F getestet hatten, welche Dexpanthenol in einer Menge von 9 Gew.-% enthielt, war im Vergleich zu den übrigen Zusammensetzung ein leicht verminderter vasokonstriktorischer Effekt zu beobachten. Dennoch war die Wirksamkeit insgesamt als gut zu bewerten.

### 3. Klinische Studie zur Analyse der Resorption von Xylometazolin:

Im Rahmen einer offenen, 2-Wege-Crossover, placebokontrollierten, randomisierten Einzeldosisstudie wurde die systemische Resorption von Xylometazolin nach topischer intranasaler Applikation in An- bzw. Abwesenheit von Dexpanthenol untersucht.

In diesem Zusammenhang wurde eine Probandengruppe mit 108 Personen im Alter von 18 bis 55 Jahren herangezogen, von denen 52 weiblich und 56 männlich waren. Der Body-Mass-Index (BMI) der Probanden lag zwischen 19 und 28 kg/m². Die Probanden befanden sich vorschriftsmäßig in einem guten gesundheitlichen Zustand. Im Rahmen der klinischen Studie wurden die 108 Probanden auf 9 Untergruppen von jeweils 12 Probanden aufgeteilt, welche jeweils eine der zuvor beschriebenen Zusammensetzungen zu Analysezwecken erhielten. Die Vorgehensweise im Rahmen der klinischen Studie wird nachfolgend beschrieben.

Wie zuvor erwähnt, wurden die Versuche im Rahmen einer offenen, blockrandomisierten, 2-Wege-Crossover-, placebokontrollierten Einzeldosisstudie mit zwei Perioden bzw. zwei Sequenzen durchgeführt. Die Probanden wurden bis zu einem Zeitpunkt von 24 Stunden nach der Applikation der zu testenden Zusammensetzung untersucht bzw. klinisch überwacht. Die Vorgehensweise im Rahmen der Studie war derart, dass die Probanden im Rahmen der ersten Periode der klinischen Studie zum Zeitpunkt 0 eine Einzeldosis (d. h. pro Nasenloch einen Sprühstoß von jeweils 75 bis 125 µl) der Testzusammensetzung erhielten. Vor Applikation der Zusammensetzung (Zeitpunkt 0) wurden den Probanden zwei Blutproben von 5 ml und 20 ml entnommen. Zu den Zeitpunkten 5 min, 10 min, 15 min und 0,5, 1,0, 1,5, 2,0, 2,5, 3,0, 3,5, 4,0, 5,0, 6,0, 8,0, 12,0 und 24,0 h nach Applikation der jeweiligen Testzusammensetzung wurden Blutproben von jeweils 5 ml zur Analyse entnommen. Die Auswaschungszeit ab dem Applikationszeitpunkt betrug fünf Tage. Im Anschluss erfolgte die zweite Periode, im Rahmen derer erneut eine Einzeldosis der jeweiligen Zusammensetzung verabreicht wurde. Die Entnahme von Blutproben erfolgte äquivalent zur ersten Periode. Insgesamt wurden somit zwei Applikationsperioden mit jeder Probandengruppe durchgeführt. In den Blutproben wurde mittels HPLC-MS/MS die Konzentration von Xylometazolin bestimmt. Darüber hinaus wurden die pharmakokinetischen Parameter der maximalen systemischen Konzentration von Xylometazolin (Cₘₐₓ) im Blut sowie der pharmakokinetische Parameter AUC₀₋ₜ bestimmt, welcher die Fläche unterhalb der integrierten Konzentrationskurve von Xylometazolin im Plasma zwischen dem Zeitpunkt t₀ und der letzten analysierten Probe (t₂₄ₕ) darstellt. Die Ergebnisse sind in der nachfolgenden Tabelle dargestellt, wobei geringe Werte der Parameter Cₘₐₓ und AUC₀₋ₜ ein Indikator für eine erfindungsgemäße gewünschte, verringerte systemische Resorption des Xylometazolins sind:

**Tabelle: Ergebnisse der klinischen Studie hinsichtlich der systemischen Resorption von Xylometazolin (Hydrochloridform)**

| **Rezeptur** | **A** | **B** | **C** | **D** | **E** | **F** | **G** | **H** | **I** |
|---|---|---|---|---|---|---|---|---|---|
| Cₘₐₓ [pg/ml] | 62,7 | 60,3 | 59,9 | 56,4 | 55,4 | 52,2 | 57,3 | 56,9 | 55,2 |
| AUC₀₋ₜ | 629,3 | 580,2 | 490,0 | 424,5 | 422,1 | 419,3 | 426,3 | 427,2 | 420,9 |

Zur statistischen Auswertung von Cₘₐₓ bzw. AUC₀₋ₜ wurde zunächst eine logarithmische Transformation der gemessenen Werte durchgeführt. Anschließend wurden die vorgenannten pharmakokinetischen Parameter mittels Varianzanalyse ermittelt.

Die vorstehenden Ergebnisse in Bezug auf die gemessenen und statistisch ausgewerteten Mengen an Xylometazolin im Blut, d. h. in Bezug auf die systemische Resorption von Xylometazolin, zeigen, dass durch den zielgerichteten Einsatz von Dexpanthenol in definierten Mengen in signifikanter Weise die systemisch aufgenommene Menge an Xylometazolin gesenkt werden kann.

Als optimal hat sich in diesem Zusammenhang eine Dexpanthenolmenge von 5,0 Gew.-% erwiesen (Rezeptur D). Bei Einsatz von 5,0 Gew.-% Dexpanthenol werden gegenüber dem nichterfindungsgemäßen Einsatz von 0 Gew.-% Dexpanthenol (Rezeptur A) 33 % weniger Xylometazolin, bezogen auf die im Probenzeitraum gemessene Gesamtmenge von Xylometazolin im Blut, resorbiert. Darüber hinaus liegt die maximale Xylometazolinkonzentration bei Einsatz von 5 Gew.-% Dexpanthenol mehr als 10 % unterhalb der maximalen Xylometazolinkonzentration ohne den Einsatz von Dexpanthenol. Durch den Einsatz von Dexpanthenol in einer Menge von 5 Gew.-% wird die Wirksamkeit des Xylometazolins, d. h. die vasokonstriktorische Wirkung, nicht beeinträchtigt, wie insbesondere auch die in Abschnitt 2.) beschriebenen Ergebnisse zeigen.

Bei Einsatz von nur 0,1 Gew.-% Dexpanthenol (Rezeptur B) steigt die systemisch resorbierte Menge an Xylometazolin im Blut an, ist jedoch trotzdem weitaus geringer als ohne den Einsatz von Dexpanthenol.

Der Einsatz von 9,0 Gew.-% Dexpanthenol (Rezeptur F) führt nur noch zu einem leichten Rückgang der systemischen Resorption von Xylometazolin im Vergleich zu dem Einsatz von 5,0 Gew.-% oder 6,0 Gew.-% Dexpanthenol, wobei jedoch die vasokonstriktorische Wirkung des Xylometazolins durch den Einsatz von Dexpanthenol in einer Menge von 9,0 Gew.-% leicht beeinträchtigt wird (vgl. Ergebnisse in Abschnitt 2.)).

Unter Berücksichtigung aller relevanten Faktoren - also Wirksamkeit einerseits sowie Verminderung der Resorption andererseits - scheinen Dexpanthenol-Mengen im Bereich von 3,0 Gew.-% bis 6,0 Gew.-% optimal zu sein.

Darüber hinaus hat sich ein pH-Wert im Bereich von 5,2 bis 5,9 als optimal erwiesen. Wie die Ergebnisse im Zusammenhang mit den Rezepturen G und H zeigen, werden jedoch auch bei Einsatz eines geringeren bzw. höheren pH-Wertes von 5,0 bzw. 6,2 gute Ergebnisse erzielt.

Durch den Einsatz von Benzalkoniumchlorid als Konservierungs- bzw. Desinfektionsmittel (Rezeptur I) lassen sich die Eigenschaften der Zusammensetzungen für die intranasale Applikation noch weitergehend verbessern, wie den obigen Ergebnissen zu entnehmen ist.

Insgesamt zeigen die vorstehend referierten Ergebnisse, dass Dexpanthenol in vollkommen überraschender Weise einen signifikanten Einfluss auf die systemische Resorption von Xylometazolin bei intranasaler Applikation besitzt. Im Rahmen der vorliegenden Erfindung ist also in überraschender Weise ein neuartiger Ansatzpunkt mit eigener Optimierung gefunden worden, welcher zu einer Eingrenzung bzw. Linderung der gravierenden Nebenwirkungen von Xylometazolin führt. Darüber hinaus wird die pharmazeutische Wirkung, d. h. der vasokonstriktorische Effekt von Xylometazolin bzw. der alpha-Sympathomimetika, durch den zusätzlichen Einsatz von Dexpanthenol speziell in den zur Linderung der systemischen Applikation ausgewählten Mengenbereichen nicht beeinträchtigt.

## Patentansprüche

1. Kombinationstherapeutikum in Form einer pharmazeutischen Zusammensetzung zur Verwendung bei der topischen nasalen Behandlung von Rhinitiden zu Zwecken der Verringerung der systemischen Resorption imidazolinbasierter alpha-Sympathomimetika, wobei die pharmazeutische Zusammensetzung in Kombination und jeweils in pharmazeutisch wirksamen Mengen
(a)
(a1) Pantothenol (Dexpanthenol) oder dessen physiologisch unbedenkliche Ester und/oder
(a2) Pantothensäure oder deren physiologisch unbedenkliche Salze;
und
(b) mindestens ein imidazolinbasiertes alpha-Sympathomimetikum oder dessen physiologisch unbedenkliches Salz;
enthält.

2. Kombinationstherapeutikum nach Anspruch 1 zur Verwendung nach Anspruch 1,
wobei die Komponente (a) aus Pantothenol (Dexpanthenol) oder dessen physiologisch unbedenklichen Estern ausgewählt ist und vorzugsweise Pantothenol (Dexpanthenol) ist; und/oder
wobei die Komponente (b) und/oder das imidazolinbasierte alpha-Sympathomimetikum ausgewählt ist aus Xylometazolin oder Oxymetazolin, insbesondere in Form von deren physiologisch unbedenklichen Salzen, besonders bevorzugt in Form von deren Hydrochloridsalzen; und/oder
wobei die Komponente (b) und/oder das imidazolinbasierte alpha-Sympathomimetikum aus Xylometazolin ist, insbesondere in Form von dessen physiologisch unbedenklichen Salzen, besonders bevorzugt in Form von dessen Hydrochloridsalz (Xylometazolinhydrochlorid).

3. Kombinationstherapeutikum nach Anspruch 1 oder 2 zur Verwendung nach Anspruch 1 oder 2,
wobei die Komponente (a), vorzugsweise in Form von Pantothenol (Dexpanthenol), mit einer Einzeldosis im Bereich von 0,1 mg bis 50 mg, insbesondere im Bereich von 0,5 mg bis 25 mg, vorzugsweise im Bereich von 1 mg bis 20 mg, bevorzugt im Bereich von 2,5 mg bis 10 mg, besonders bevorzugt im Bereich von 4 mg bis 8 mg, verabreicht wird bzw. wobei die Komponente (a), vorzugsweise in Form von Pantothenol (Dexpanthenol), zur Verabreichung mit einer Einzeldosis im Bereich von 0,1 mg bis 50 mg, insbesondere im Bereich von 0,5 mg bis 25 mg, vorzugsweise im Bereich von 1 mg bis 20 mg, bevorzugt im Bereich von 2,5 mg bis 10 mg, besonders bevorzugt im Bereich von 4 mg bis 8 mg, hergerichtet ist, insbesondere wobei 2 bis 10 Einzeldosen pro Tag, insbesondere 3 bis 5 Einzeldosen pro Tag, verabreicht werden, vorzugsweise über den Tag verteilt, und/oder insbesondere wobei die Komponente (a), vorzugsweise in Form von Pantothenol (Dexpanthenol), zur Verabreichung in 2 bis 10 Einzeldosen pro Tag, insbesondere 3 bis 5 Einzeldosen pro Tag, hergerichtet ist, vorzugsweise über den Tag verteilt; und/oder
wobei die Komponente (b), vorzugsweise in Form von Xylometazolin und/oder Oxymetazolin oder deren physiologisch unbedenklichen Salzen, besonders bevorzugt in Form von Xylometazolinhydochlorid, mit einer Einzeldosis im Bereich von 0,001 mg bis 10 mg, insbesondere im Bereich von 0,01 mg bis 5 mg, vorzugsweise im Bereich von 0,05 mg bis 2 mg, bevorzugt im Bereich von 0,08 mg bis 1 mg, besonders bevorzugt im Bereich von 0,1 mg bis 0,8 mg, verabreicht wird bzw. wobei die Komponente (b), vorzugsweise in Form von Xylometazolin und/oder Oxymetazolin oder deren physiologisch unbedenklichen Salzen, besonders bevorzugt in Form von Xylometazolinhydochlorid, zur Verabreichung mit einer Einzeldosis im Bereich von 0,001 mg bis 10 mg, insbesondere im Bereich von 0,01 mg bis 5 mg, vorzugsweise im Bereich von 0,05 mg bis 2 mg, bevorzugt im Bereich von 0,08 mg bis 1 mg, besonders bevorzugt im Bereich von 0,1 mg bis 0,8 mg, hergerichtet ist, insbesondere wobei 2 bis 10 Einzeldosen pro Tag, insbesondere 3 bis 5 Einzeldosen pro Tag, verabreicht werden, vorzugsweise über den Tag verteilt, und/oder insbesondere wobei die Komponente (b), vorzugsweise in Form von Xylometazolin und/oder Oxymetazolin oder deren physiologisch unbedenklichen Salzen, besonders bevorzugt in Form von Xylometazolinhydochlorid, zur Verabreichung in 2 bis 10 Einzeldosen pro Tag, insbesondere 3 bis 5 Einzeldosen pro Tag, hergerichtet ist, vorzugsweise über den Tag verteilt; und/oder
wobei die Komponente (a) zusammen und/oder in Kombination mit der Komponente (b) in einem Gewichtsverhältnis von Komponente (a) zu Komponente (b) im Bereich von 10 : 1 bis 1.000 : 1, insbesondere 15 : 1 bis 500 : 1, vorzugsweise 20 : 1 bis 250 : 1, bevorzugt 25 : 1 bis 200 : 1, besonders bevorzugt 30 : 1 bis 175 : 1, ganz besonders bevorzugt 40 : 1 bis 150 : 1, noch mehr bevorzugt 45: 1 bis 125 : 1, eingesetzt wird und/oder wobei die Komponente (a) zusammen und/oder in Kombination mit der Komponente (b) zur Verabreichung in einem Gewichtsverhältnis von Komponente (a) zu Komponente (b) im Bereich von 10 : 1 bis 1.000 : 1, insbesondere 15 : 1 bis 500 : 1, vorzugsweise 20 : 1 bis 250 : 1, bevorzugt 25 : 1 bis 200 : 1, besonders bevorzugt 30 : 1 bis 175 : 1, ganz besonders bevorzugt 40 : 1 bis 150 : 1, noch mehr bevorzugt 45 : 1 bis 125 : 1, hergerichtet ist.

4. Kombinationstherapeutikum nach einem der vorangehenden Ansprüche zur Verwendung nach einem der vorangehenden Ansprüche,
wobei die Komponente (a) in der pharmazeutischen Zusammensetzung inkorporiert ist, wobei die Zusammensetzung, bezogen auf die Zusammensetzung, die Komponente (a) in einer Menge von 0,01 bis 10 Gew.-%, insbesondere 0,1 bis 9 Gew.-%, vorzugsweise 0,5 bis 8 Gew.-%, bevorzugt 1 bis 7 Gew.-%, besonders bevorzugt 2 bis 6 Gew.-%, ganz besonders bevorzugt 3 bis 6 Gew.-%, enthält; und/oder
wobei die Komponente (b) in der pharmazeutischen Zusammensetzung inkorporiert ist, wobei die Zusammensetzung, bezogen auf die Zusammensetzung, die Komponente (b) in einer Menge von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthält; und/oder
wobei die Komponente (a) zusammen und/oder in Kombination mit der Komponente (b) als wässriges System, insbesondere als wässriges Einphasensystem, vorzugsweise als wässrige Lösung oder wässrige Solubilisierung, eingesetzt ist und/oder zur Verabreichung hergerichtet ist und/oder wobei die Komponente (a) zusammen und/oder in Kombination mit der Komponente (b) mit einem Exzipienten oder Träger auf Wasserbasis eingesetzt ist und/oder zur Verabreichung hergerichtet ist und/oder wobei die Komponente (a) zusammen und/oder in Kombination mit der Komponente (b) als klare, farblose wässrige Lösung eingesetzt ist und/oder zur Verabreichung hergerichtet ist.

5. Kombinationstherapeutikum nach einem der vorangehenden Ansprüche zur Verwendung nach einem der vorangehenden Ansprüche,
wobei die Komponente (a) zusammen und/oder in Kombination mit der Komponente (b) in der pharmazeutischen Zusammensetzung mit einem pH-Wert im Bereich von 5,0 bis 6,2, insbesondere im Bereich von 5,0 bis 6,0, vorzugsweise im Bereich von 5,1 bis 6,0, bevorzugt im Bereich von 5,2 bis 5,9, eingesetzt ist und/oder zur Verabreichung hergerichtet ist;
insbesondere wobei zur Einstellung und/oder Konstanthaltung des pH-Werts der pharmazeutischen Zusammensetzung mindestens ein chemisches Puffersystem, insbesondere Puffersalz(e), eingesetzt ist, wobei als chemisches Puffersystem bevorzugt ein Dihydrogenphosphat/Monohydrogenphosphat-Puffersystem ("H₂PO₄⁻/HPO₄²⁻-Puffer(system)" bzw. "Phosphatpuffer(system)"), insbesondere ein Alkalidihydrogenphosphat/Alkalimonohydrogenphosphat-Puffersystem, eingesetzt ist, insbesondere wobei das Dihydrogenphosphat/Monohydrogenphosphat-Puffersystem mit einem Dihydrogenphosphat/Monohydrogenphosphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 100 : 1, eingesetzt ist.

6. Kombinationstherapeutikum nach einem der vorangehenden Ansprüche zur Verwendung nach einem der vorangehenden Ansprüche,
wobei die Komponenten (a) und (b) zur Verabreichung zusammen und/oder in Kombination mit (c) mindestens einem Konservierungsmittel und/oder Desinfektionsmittel hergerichtet sind, wobei die Komponenten (a), (b) und (c) in der pharmazeutischen Zusammensetzung oder einem Arzneimittel, inkorporiert sind bzw. vorliegen;
insbesondere wobei das Konservierungsmittel und/oder Desinfektionsmittel ausgewählt ist aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) para-Hydroxybenzoesäureestern und Mischungen von verschiedenen para-Hydroxybenzoesäureestern, vorzugsweise Nipa-Estern, (iii) Chlorhexidin sowie Kombinationen der vorgenannten Verbindungen und besonders bevorzugt Benzalkoniumchlorid ist; und/oder
insbesondere wobei die Komponente (c), vorzugsweise in Form von Benzalkoniumchlorid, mit einer Einzeldosis im Bereich von 0,001 mg bis 5 mg, insbesondere im Bereich von 0,005 mg bis 2 mg, vorzugsweise im Bereich von 0,01 mg bis 1 mg, verabreicht wird bzw. wobei die Komponente (c), vorzugsweise in Form von Benzalkoniumchlorid, zur Verabreichung mit einer Einzeldosis im Bereich von 0,005 mg bis 2 mg, vorzugsweise im Bereich von 0,01 mg bis 1 mg, hergerichtet ist, insbesondere wobei 2 bis 10 Einzeldosen pro Tag, insbesondere 3 bis 5 Einzeldosen pro Tag, verabreicht werden, vorzugsweise über den Tag verteilt, und/oder insbesondere wobei die Komponente (c), vorzugsweise in Form von Benzalkoniumchlorid, zur Verabreichung in 2 bis 10 Einzeldosen pro Tag, insbesondere 3 bis 5 Einzeldosen pro Tag, hergerichtet ist, vorzugsweise über den Tag verteilt; und/oder
insbesondere wobei die Komponente (c) in der pharmazeutischen Zusammensetzung inkorporiert ist, wobei die Zusammensetzung, bezogen auf die Zusammensetzung, die Komponente (c) in einer Menge von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, enthält.

7. Kombinationstherapeutikum nach einem der vorangehenden Ansprüche zur Verwendung nach einem der vorangehenden Ansprüche,
wobei die Komponenten (a) und (b) und gegebenenfalls (c) zur Verabreichung zusammen und/oder in Kombination mit (d) mindestens einem vorzugsweise sauren Glykosaminoglykan oder dessen physiologisch unbedenklichen Salzen oder Derivaten, insbesondere Hyaluronsäure oder deren physiologisch unbedenklichen Salzen, hergerichtet sind, wobei die Komponenten (a), (b), (c) und (d) in der pharmazeutischen Zusammensetzung inkorporiert sind bzw. vorliegen;
insbesondere wobei die Komponente (d) in der pharmazeutischen Zusammensetzung inkorporiert ist, wobei die Zusammensetzung, bezogen auf die Zusammensetzung, die Komponente (d) in einer Menge von 0,0001 bis 10 Gew.-%, insbesondere 0,001 bis 5 Gew.-%, vorzugsweise 0,01 bis 2 Gew.-%, enthält.

8. Kombinationstherapeutikum nach einem der vorangehenden Ansprüche zur Verwendung nach einem der vorangehenden Ansprüche,
wobei die Komponenten (a) und (b) und gegebenenfalls (c) und/oder (d) zur Verabreichung zusammen und/oder in Kombination mit (e) Ectoin oder mindestens einem Ectoinderivat, insbesondere einem Hydroxyectoin, hergerichtet sind, wobei die Komponenten (a), (b), (c), (d) und (e) in der pharmazeutischen Zusammensetzung inkorporiert sind bzw. vorliegen;
insbesondere wobei die Komponente (e) in der pharmazeutischen Zusammensetzung inkorporiert ist, wobei die Zusammensetzung, bezogen auf die Zusammensetzung, die Komponente (e) in einer Menge von 0,0001 bis 10 Gew.-%, insbesondere 0,001 bis 5 Gew.-%, vorzugsweise 0,01 bis 2 Gew.-%, enthält.

9. Kombinationstherapeutikum nach einem der vorangehenden Ansprüche zur Verwendung nach einem der vorangehenden Ansprüche,
wobei die Komponenten (a) und (b) und gegebenenfalls (c), (d) und/oder (e) zur Verabreichung zusammen und/oder in Kombination mit (f) Natriumchlorid hergerichtet sind, insbesondere wobei die Komponenten (a), (b), (c), (d), (e) und (f) in der pharmazeutischen Zusammensetzung inkorporiert sind bzw. vorliegen;
insbesondere wobei die Komponente (f) in der pharmazeutischen Zusammensetzung inkorporiert ist, wobei die Zusammensetzung, bezogen auf die Zusammensetzung, die Komponente (e) in einer Menge von 0,001 bis 5 Gew.-%, insbesondere 0,01 bis 2 Gew.-%, vorzugsweise 0,1 bis 1 Gew.-%, enthält.

10. Kombinationstherapeutikum nach einem der vorangehenden Ansprüche zur Verwendung nach einem der vorangehenden Ansprüche, wobei alle Wirk- und/oder Inhaltsstoffe, insbesondere die Komponenten (a) und (b) und gegebenenfalls (c), (d), (e) und/oder (f), in der pharmazeutischen Zusammensetzung inkorporiert sind, wobei die Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist.

11. Kombinationstherapeutikum nach einem der vorangehenden Ansprüche zur Verwendung nach einem der vorangehenden Ansprüche,
wobei alle Wirk- und/oder Inhaltsstoffe, insbesondere die Komponenten (a) und (b) und gegebenenfalls (c), (d), (e) und/oder (f), in der pharmazeutischen Zusammensetzung inkorporiert sind, wobei die Zusammensetzung bei einer Temperatur von 20 °C und bei einem Druck von 1.013,25 mbar eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist; und/oder
wobei alle Wirk- und/oder Inhaltsstoffe, insbesondere die Komponenten (a) und (b) und gegebenenfalls (c), (d), (e) und/oder (f), in der pharmazeutischen Zusammensetzung inkorporiert sind, wobei die Zusammensetzung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil, ist.

12. Kombinationstherapeutikum nach einem der vorangehenden Ansprüche zur Verwendung nach einem der vorangehenden Ansprüche,
wobei alle Wirk- und/oder Inhaltsstoffe, insbesondere die Komponenten (a) und (b) und gegebenenfalls (c), (d), (e) und/oder (f), in der pharmazeutischen Zusammensetzung inkorporiert sind, wobei die Zusammensetzung einen Gehalt an Abbauprodukt(en) der Komponente (b), insbesondere Verunreinigung A, von höchstens 5 Gew.-%, insbesondere von höchstens 3 Gew.-%, vorzugsweise von höchstens 2 Gew.-%, besonders bevorzugt von höchstens 1 Gew.-%, bezogen auf den Wirkstoff (b), aufweist, insbesondere auch nach Lagerung des Arzneimittels bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, und/oder
wobei alle Wirk- und/oder Inhaltsstoffe, insbesondere die Komponenten (a) und (b) und gegebenenfalls (c), (d), (e) und/oder (f), in **der** pharmazeutischen Zusammensetzung inkorporiert sind, wobei die Zusammensetzung einen Gehalt an Abbauprodukt(en) der Komponente (a), insbesondere Aminopropanol und/oder D-Pantolacton, von jeweils höchstens 5 Gew.-%, insbesondere von jeweils höchstens 3 Gew.-%, vorzugsweise von jeweils höchstens 2 Gew.-%, besonders bevorzugt von jeweils höchstens 1 Gew.-%, bezogen auf den Wirkstoff (a), aufweist, insbesondere auch nach Lagerung des Arzneimittels bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten.

13. Kombinationstherapeutikum nach einem der vorangehenden Ansprüche zur Verwendung bei der Behandlung von Rhinitiden aller Art, insbesondere *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta;* und/oder
zur Verwendung bei der prophylaktischen und/oder kurativen topischen Behandlung von Rhinitiden, insbesondere *Rhinitis acuta.*

14. Kombinationstherapeutikum nach einem der vorangehenden Ansprüche zur Verwendung nach einem der vorangehenden Ansprüche,
wobei alle Wirk- und/oder Inhaltsstoffe, insbesondere die Komponenten (a) und (b) und gegebenenfalls (c), (d), (e) und/oder (f), in der pharmazeutischen Zusammensetzung inkorporiert sind, wobei die Zusammensetzung mittels einer Applikationsvorrichtung, bevorzugt in Form eines Behältnisses mit Tropf- oder Sprüheinrichtung, verabreicht wird und/oder zur Verabreichung hergerichtet ist; insbesondere wobei die Applikationsvorrichtung eine Sprüheinrichtung zur gleichförmigen Ausbringung der Zusammensetzung in einer Menge pro Sprühstoß im Bereich von 25 µl bis 300 µl, insbesondere im Bereich von 50 µl bis 200 µl, vorzugsweise im Bereich von 75 µl bis 125 µl, aufweist; und/oder wobei die Applikationsvorrichtung einen Vorratsbehälter mit einem Volumen im Bereich von 5 ml bis 100 ml, insbesondere 10 ml bis 50 ml, aufweist.

15. Pantothenol (Dexpanthenol) oder dessen physiologisch unbedenkliche Ester und/oder Pantothensäure oder deren physiologisch unbedenkliche Salze zur Verwendung bei der prophylaktischen und/oder kurativen Behandlung von Rhinitiden mittels nasaler Applikation zu Zwecken der Verringerung der systemischen Resorption eines imidazolinbasierten alpha-Sympathomimetikums, wobei das Pantothenol (Dexpanthenol) oder dessen physiologisch unbedenkliche Ester und/oder die Pantothensäure oder deren physiologisch unbedenkliche Salze zusammen und/oder in Kombination mit dem imidazolinbasierten alpha-Sympathomimetikum oder dessen physiologisch unbedenklichen Salzen verabreicht wird.

## Claims

1. Combination therapeutic agent in the form of a pharmaceutical composition for use in the topical treatment of nasal rhinitis for purposes of reducing the systemic absorption of imidazoline-based alpha-sympathomimetic substances,
wherein the pharmaceutical composition contains respectively in combination and in pharmaceutically effective amounts
(a) (a1) pantothenol (dexpanthenol) or its physiologically acceptable esters and/or (a2) pantothenic acid or its physiologically acceptable salts;
and
(b) at least one imidazoline-based alpha-sympathomimetic substance or its physiologically acceptable salts.

2. Combination therapeutic agent according to claim 1 and for use according to claim 1, wherein component (a) is selected from pantothenol (dexpanthenol) or its physiologically acceptable esters thereof and preferably pantothenol (dexpanthenol); and/or
wherein component (b) and/or the imidazoline-based alpha-sympathomimetic substance is selected from xylometazoline or oxymetazoline, in particular in the form of their physiologically acceptable salts, more preferably in the form of their hydrochloride salts; and/or
wherein component (b) and/or the imidazoline-based alpha-sympathomimetic substance is selected from xylometazoline, in particular in the form of its physiologically acceptable salts, more preferably in the form of its hydrochloride salt (xylometazoline hydrochloride).

3. Combination therapeutic agent according to claim 1 or 2 and for use according to claim 1 or 2,
wherein component (a) is administered preferably in the form of pantothenol (dexpanthenol) with a single dose ranging from 0.1 mg to 50 mg, in particular in the range from 0.5 mg to 25 mg, preferably in the range from 1 mg to 20 mg, more preferably in the range from 2.5 mg to 10 mg, particularly preferably in the range from 4 mg to 8 mg, or wherein component (a) is administered preferably in the form of pantothenol (dexpanthenol) with a single dose ranging from 0.1 mg to 50 mg, in particular in the range from 0.5 mg to 25 mg, preferably in the range of 1 mg to 20 mg, more preferably in the range from 2.5 mg to 10 mg, particularly preferably in the range from 4 mg to 8 mg, in particular wherein from 2 to 10 individual doses may be administered per day, in particular in 3 to 5 individual doses per day, preferably spread over the day, and/or in particular wherein component (a) is administered preferably in the form of pantothenol (dexpanthenol) in 2 to 10 individual doses per day, in particular in 3 to 5 individual doses per day, preferably spread over the day; and/or
wherein component (b) is administered preferably in the form of xylometazoline and/or oxymetazoline, or its physiologically acceptable salts, more preferably in the form of xylometazoline hydrochloride, with a single dose ranging from 0.001 mg to 10 mg, in particular from 0.01 mg to 5 mg, preferably in the range from 0.05 mg to 2 mg, preferably in the range of 0.08 mg to 1 mg, particularly preferably in the range of 0.1 mg to 0.8 mg, and wherein component (b) is administered preferably in the form of xylometazoline and/or oxymetazoline, or their physiologically acceptable salts, more preferably in the form of xylometazoline hydrochloride with a unit dose in the range from 0.001 mg to 10 mg, in particular from 0.01 mg to 5 mg , preferably in the range from 0.05 mg to 2 mg, more preferably in the range from 0.08 mg to 1 mg, particularly preferably in the range from 0.1 mg to 0.8 mg, in particular wherein from 2 to 10 individual doses may be administered per day, especially 3 to 5 individual doses per day, preferably spread over the day, and/or in particular wherein component (b) is administered preferably in the form of xylometazoline and/or oxymetazoline, or their physiologically acceptable salts, more preferably in the form of xylometazoline hydrochloride in 2 to 10 individual doses per day, in particular 3 to 5 individual doses per day, preferably throughout the day; and/or
wherein component (a) together with and/or in combination with component (b) in a weight ratio of component (a) to component (b) range from 10 : 1 to 1,000 : 1, especially 15 : 1 to 500 : 1, preferably 20 : 1 to 250 : 1, more preferably 25 : 1 to 200 : 1, most preferably 30 : 1 to 175 : 1, particularly preferably 40 : 1 to 150 : 1, even more preferably 45 : 1 to 125 : 1 is used, and/or wherein component (a) together with and/or in combination with component (b) is administered in a weight ratio of component (a) to component (b) range from 10 : 1 to 1,000 : 1, in particular from 15 : 1 to 500 : 1, preferably 20 : 1 to 250 : 1, more preferably 25 : 1 to 200 : 1, particularly preferably 30 : 1 to 175 : 1, most preferably 40 : 1 to 150 : 1, even more preferably 45 : 1 to 125 : 1.

4. Combination therapeutic agent according to one of the preceding claims and for use according to one of the preceding claims,
wherein component (a) is incorporated in the pharmaceutical composition,
wherein the composition, based on the composition, contains component (a) in an amount of 0.01 to 10 wt .-%, particularly 0.1 to 9 wt %, preferably 0.5 to 8 wt .-%, more preferably 1 to 7 wt.-%, particularly preferably 2 to 6 wt.-%, most preferably 3 to 6 wt.-%; and/or
wherein component (b) is incorporated in the pharmaceutical composition, wherein the composition, based on the composition, contains component (b) in an amount of 0.001 to 2 wt .-%, particularly 0.005 to 1.5 wt .-%, preferably 0.01 to 1.2 wt .-%, more preferably 0.02 to 1.0 wt.-%, particularly preferably 0.03 to 0.5 wt .-%, most preferably 0.04 to 0.2 wt.-%; and/or
wherein component (a) together with and/or in combination with component (b) is used as an aqueous system, in particular as an aqueous single-phase system, preferably as an aqueous solution or aqueous solubilisation and/or is prepared for administration, and/or wherein component (a) is used together with and/or in combination with component (b) with a water-based excipient or carrier, and/or is prepared for administration and/or wherein component (a) together with and/or in combination with component (b) is used as a clear colourless aqueous solution and/or is prepared for administration.

5. Combination therapeutic agent according to one of the preceding claims and for use according to one of the preceding claims,
wherein component (a) together with and/or, in combination with component (b) is used and/or prepared for administration in the pharmaceutical composition with a pH value in the range of 5.0 to 6.2, in particular in the range from 5.0 to 6.0, preferably in the range of 5.1 to 6.0, more preferably in the range from 5.2 to 5.9;
in particular wherein the adjustment and/or maintenance of the pH value of the pharmaceutical composition of at least one chemical buffer system uses, in particular, buffer salt(s), wherein preferably a dihydrogen phosphate/disodium phosphate buffer system ("H₂PO₄⁻/HPO₄²⁻ - buffer (system) "and" phosphate buffer (system)"), wherein particularly an alkali dihydrogen phosphate/alkali monohydrogen phosphate buffer system is used as a chemical buffer system, in particular wherein the dihydrogen phosphate/monohydrogen phosphate buffer system is used with a dihydrogen phosphate/disodium phosphate molar ratio from greater than 5 : 1, particularly in the range from 5 : 1 to 110 : 1, more preferably in the range from 6 : 1 to 105 : 1, most preferably in the range from 7 : 1 to 100 : 1.

6. Combination therapeutic agent according to one of the preceding claims and for use according to one of the preceding claims,
wherein, for administration, components (a) and (b) together with and/or in combination with (c) are prepared with at least one preserving agent and/or disinfecting agent, in particular wherein components (a), (b) and (c) are incorporated or are present in the pharmaceutical composition or a drug;
in particular wherein the preserving agent and/or disinfecting agent is selected from the group consisting of (i) alkylbenzyldimethylammonium chlorides, in particular C₈-C₁₈-alkylbenzyldimethylammonium chlorides, and mixtures of various alkylbenzyldimethylammonium chlorides, preferably benzalkonium chloride, (ii) para-hydroxybenzoic acid and mixtures of various para-hydroxybenzoic acid esters, preferably from Nipa esters, (iii) chlorhexidine as well as combinations of the aforementioned compounds, and most preferably benzalkonium chloride; and/or
in particular wherein component (c) is administered preferably in the form of benzalkonium chloride, with a single dose ranging from 0.001 mg to 5 mg, in particular in the range from 0.005 mg to 2 mg, preferably in the range from 0.01 mg to 1 mg or wherein component (c) is administered preferably in the form of benzalkonium chloride, with a unit dose in the range from 0.005 mg to 2 mg, preferably in the range from 0.01 mg to 1 mg, and may be administered in particular in 2 to 10 individual doses per day, in particular 3 to 5 individual doses per day, preferably spread over the day, and/or in particular wherein component (c) is administered preferably in the form of benzalkonium chloride in 2 to 10 individual doses per day, in particular 3 to 5 individual doses per day, preferably spread over the day; and/or
in particular wherein component (c) is incorporated in the pharmaceutical composition, wherein the composition, based on the composition, contains component (c) in an amount from 0.001 to 10 wt.-%, in particular from 0.005 to 5 wt.-%, preferably 0.01 to 2 wt.-%, more preferably 0.01 to 1 wt.-%, particularly preferably 0.01 to 0.5 wt.-%.

7. Combination therapeutic agent according to one of the preceding claims and for use according to one of the preceding claims,
wherein components (a) and (b) and optionally (c) are prepared for administration together and/or in combination with (d) at least one preferably acidic glycosaminoglycan or its physiologically acceptable salts or derivatives thereof, in particular hyaluronic acid or its physiologically acceptable salts, wherein components (a), (b), (c) and (d) are incorporated or are present in the pharmaceutical composition;
in particular wherein component (d) is incorporated in the pharmaceutical composition, wherein the composition, based on the composition, contains component (d) in an amount of 0.0001 to 10 wt.-%, in particular 0.001 to 5 wt.-% , preferably 0.01 to 2 wt.-%.

8. Combination therapeutic agent according to one of the preceding claims and for use according to one of the preceding claims,
wherein components (a) and (b) and optionally (c) and/or (d) are prepared for administration together with and/or in combination with (e) ectoin or at least one ectoin derivative, in particular a hydroxyectoine, wherein components (a), (b), (c), (d) and (e) are incorporated or are present in the pharmaceutical composition;
in particular wherein component (e) is incorporated in the pharmaceutical composition, wherein the composition, based on the composition, contains component (e) in an amount of 0.0001 to 10 wt.-%, in particular 0.001 to 5 wt.-%, preferably 0.01 to 2 wt.-%.

9. Combination therapeutic agent according to one of the preceding claims and for use according to one of the preceding claims,
wherein components (a) and (b) and optionally (c), (d) and/or (e) are prepared for administration together with and/or in combination with (f) sodium chloride, in particular wherein the components (a), (b), (c), (d), (e) and (f) are incorporated or are present in the pharmaceutical composition;
in particular wherein component (f) is incorporated in the pharmaceutical composition, wherein the composition, based on the composition, contains component (e) in an amount of 0.001 to 5 wt.-%, in particular 0.01 to 2 wt.%, preferably 0.1 to 1 wt.-%.

10. Combination therapeutic agent according to one of the preceding claims and used according to any one of the preceding claims,
wherein all the active substances and/or ingredients, in particular components (a) and (b) and optionally (c), (d), (e) and/or (f), are incorporated in the pharmaceutical composition, wherein the said composition comprises an osmolality in the range from 300 to 600 mosm/kg, in particular in the range from 310 to 550 mosm/kg, preferably in the range from 300 to 525 mosm/kg, more preferably in the range from 325 to 510 mosm/kg, most preferably in the range from 350 to 500 mosm/kg.

11. Combination therapeutic agent according to one of the preceding claims and used according to any one of the preceding claims,
wherein all the active substances and/or ingredients, in particular components (a) and (b) and optionally (c), (d), (e) and/or (f), are incorporated in the pharmaceutical composition, wherein the composition at a temperature of 20°C and at a pressure of 1013.25 mbar has a relative density, based on pure water, in the range from 1.001 to 1.2, in particular in the range from 1.005 to 1.15, preferably in the range from 1.005 to 1.105; and/or
wherein all active substances and/or ingredients, in particular components (a) and (b) and optionally (c), (d), (e) and/or (f) are incorporated in the pharmaceutical composition, wherein the composition is stable, particularly stable in storage, at temperatures in the range of 20°C to 50°C at a pressure of 1013.25 mbar and with a relative humidity in the range from 50% to 90% for at least 6 months, especially at least 12 months, preferably at least 24 months, more preferably at least 36 months.

12. Combination therapeutic agent according to one of the preceding claims and used according to any one of the preceding claims,
wherein all the active substances and/or ingredients, in particular components (a) and (b) and optionally (c), (d), (e) and/or (f), are incorporated in the pharmaceutical composition, wherein the said composition has a content of degradation product(s) of component (b), in particular impurity A, of at most 5 wt.-%, in particular not exceeding 3 wt .-%, preferably at most 2 wt.-%, particularly preferably at most 1 wt %, based on the active substance (b) which, in particular, even after storage of the drug at temperatures in the range of 20°C to 50°C at a pressure of 1013.25 mbar and at a relative humidity in the range of 50% to 90% for at least 6 months, preferably at least 12 months, more preferably for at least 24 months, most preferably at least 36 months, and/or
wherein all the active substances and/or ingredients, in particular components (a) and (b) and optionally (c), (d), (e) and/or (f), are incorporated in the pharmaceutical composition, wherein the said composition has a content of degradation product(s) of component (a), in particular aminopropanol and/or D-pantolactone, of respectively not more than 5 wt.-%, in particular respectively at most 3 wt.-%, preferably respectively at most 2 wt.-%, particularly preferably respectively at most 1 wt.-%, based on the active substance (a), in particular, even after storage of the drug at temperatures in the range of 20°C to 50°C, at a pressure of 1013.25 mbar and at a relative humidity in the range from 50% to 90% of at least 6 months, especially at least 12 months, preferably at least 24 months, more preferably at least 36 months.

13. Combination therapeutic agent according to any one of the preceding claims for use in the treatment of rhinitis of all types, in particular, *Rhinitis acuta, Rhinitis allergica, Rhinitis atrophieans, Rhinitis hyperplastica* or *hypertrophieans, Rhinitis mutilans, Rhinitis nervosa* or *vasomotorica* or *Rhinitis pseudomembranacea,* preferably *Rhinitis acuta;* and/or for use in the prophylactic and/or curative topical treatment of rhinitis, in particular *Rhinitis acuta.*

14. Combination therapeutic agent according to any one of the preceding claims and use according to any one of the preceding claims,
wherein all the active substances and/or ingredients, in particular components (a) and (b) and optionally (c), (d), (e) and/or (f), are incorporated in the pharmaceutical composition, wherein the composition is preferably administered by means of an application device in the form of a container through dripping or spraying, and/or is prepared for administration; in particular wherein the application device is a spray device for the uniform application of the composition in an amount per shot in the range from 25 µl to 300 µl, in particular in the range from 50 µl to 200 µl, preferably in the range of 75 µl to 125 µl; and/or
wherein the application device has a reservoir with a volume in the range from 5 ml to 100 ml, preferably 10 ml to 50 ml.

15. Pantothenol (dexpanthenol) or its physiologically acceptable esters and/or pantothenic acid or its physiologically acceptable salts for use in the prophylactic and/or curative treatment of rhinitis by nasal application, for purposes of reducing the systemic absorption of an imidazoline-based alpha-sympathomimetic substance, wherein the pantothenol (dexpanthenol) or its physiologically acceptable esters and/or pantothenic acid or its physiologically acceptable salts together with and/or in combination with the imidazoline-based alpha-sympathomimetic substance substance substance or its physiologically acceptable salts is administered.

## Revendications

1. Agent thérapeutique combiné sous la forme d'une composition pharmaceutique, destiné à une utilisation pour le traitement nasal topique de rhinites dans le but de réduire la résorption systémique de sympathomimétiques alpha à base d'imidazoline,
dans lequel la composition pharmaceutique contient en combinaison et à chaque fois en quantités pharmaceutiquement efficaces
(a)
(a1) du pantothénol (dexpanthénol) ou ses esters physiologiquement inoffensifs et/ou
(a2) de l'acide pantothénique ou ses sels physiologiquement inoffensifs ; et
(b) au moins un sympathomimétique alpha à base d'imidazoline ou son sel physiologiquement inoffensif.

2. Agent thérapeutique combiné selon la revendication 1 destiné à une utilisation selon la revendication 1,
dans lequel le composant (a) est choisi parmi le pantothénol (dexpanthénol) ou ses esters physiologiquement inoffensifs et est de préférence le pantothénol (dexpanthénol) ; et/ou
dans lequel le composant (b) et/ou le sympathomimétique alpha à base d'imidazoline est choisi parmi la xylométazoline ou l'oxymétazoline, notamment sous la forme de leurs sels physiologiquement inoffensifs, de manière particulièrement préférée sous la forme de leurs sels chlorhydrate ; et/ou
dans lequel le composant (b) et/ou le sympathomimétique alpha à base d'imidazoline est la xylométazoline, notamment sous la forme de ses sels physiologiquement inoffensifs, de manière particulièrement préférée sous la forme de son sel chlorhydrate (chlorhydrate de xylométazoline).

3. Agent thérapeutique combiné selon la revendication 1 ou 2 destiné à une utilisation selon la revendication 1 ou 2,
dans lequel le composant (a), de préférence sous la forme de pantothénol (dexpanthénol), est administré en une dose individuelle dans la plage allant de 0,1 mg à 50 mg, notamment dans la plage allant de 0,5 mg à 25 mg, avantageusement dans la plage allant de 1 mg à 20 mg, de préférence dans la plage allant de 2,5 mg à 10 mg, de manière particulièrement préférée dans la plage allant de 4 mg à 8 mg, ou dans lequel le composant (a), de préférence sous la forme de pantothénol (dexpanthénol), est préparé pour l'administration en une dose individuelle dans la plage allant de 0,1 mg à 50 mg, notamment dans la plage allant de 0,5 mg à 25 mg, avantageusement dans la plage allant de 1 mg à 20 mg, de préférence dans la plage allant de 2,5 mg à 10 mg, de manière particulièrement préférée dans la plage allant de 4 mg à 8 mg, notamment dans lequel 2 à 10 doses individuelles par jour, notamment 3 à 5 doses individuelles par jour, sont administrées, de préférence réparties sur la journée, et/ou notamment dans lequel le composant (a), de préférence sous la forme de pantothénol (dexpanthénol), est préparé pour l'administration en 2 à 10 doses individuelles par jour, notamment 3 à 5 doses individuelles par jour, de préférence réparties sur la journée ; et/ou
dans lequel le composant (b), de préférence sous la forme de xylométazoline et/ou d'oxymétazoline ou leurs sels physiologiquement inoffensifs, de manière particulièrement préférée sous la forme de chlorhydrate de xylométazoline, est administré en une dose individuelle dans la plage allant de 0,001 mg à 10 mg, notamment dans la plage allant de 0,01 mg à 5 mg, avantageusement dans la plage allant de 0,05 mg à 2 mg, de préférence dans la plage allant de 0,08 mg à 1 mg, de manière particulièrement préférée dans la plage allant de 0,1 mg à 0,8 mg, ou dans lequel le composant (b), de préférence sous la forme de xylométazoline et/ou d'oxymétazoline ou leurs sels physiologiquement inoffensifs, de manière particulièrement préférée sous la forme de chlorhydrate de xylométazoline, est préparé pour l'administration en une dose individuelle dans la plage allant de 0,001 mg à 10 mg, notamment dans la plage allant de 0,01 mg à 5 mg, avantageusement dans la plage allant de 0,05 mg à 2 mg, de préférence dans la plage allant de 0,08 mg à 1 mg, de manière particulièrement préférée dans la plage allant de 0,1 mg à 0,8 mg, notamment dans lequel 2 à 10 doses individuelles par jour, notamment 3 à 5 doses individuelles par jour, sont administrées, de préférence réparties sur la journée, et/ou notamment dans lequel le composant (b), de préférence sous la forme de xylométazoline et/ou d'oxymétazoline ou leurs sels physiologiquement inoffensifs, de manière particulièrement préférée sous la forme de chlorhydrate de xylométazoline, est préparé pour l'administration en 2 à 10 doses individuelles par jour, notamment 3 à 5 doses individuelles par jour, de préférence réparties sur la journée ; et/ou
dans lequel le composant (a) est utilisé conjointement avec et/ou en combinaison avec le composant (b) en un rapport en poids entre le composant (a) et le composant (b) dans la plage allant de 10:1 à 1 000:1, notamment de 15:1 à 500:1, avantageusement de 20:1 à 250:1, de préférence de 25:1 à 200:1, de manière particulièrement préférée de 30:1 à 175:1, de manière tout particulièrement préférée de 40:1 à 150:1, de manière encore davantage préférée de 45:1 à 125:1, et/ou dans lequel le composant (a) est préparé pour l'administration conjointement avec et/ou en combinaison avec le composant (b) en un rapport en poids entre le composant (a) et le composant (b) dans la plage allant de 10:1 à 1 000:1, notamment de 15:1 à 500:1, avantageusement de 20:1 à 250:1, de préférence de 25:1 à 200:1, de manière particulièrement préférée de 30:1 à 175:1, de manière tout particulièrement préférée de 40:1 à 150:1, de manière encore davantage préférée de 45:1 à 125:1.

4. Agent thérapeutique combiné selon l'une quelconque des revendications précédentes destiné à une utilisation selon l'une quelconque des revendications précédentes,
dans lequel le composant (a) est incorporé dans la composition pharmaceutique, la composition contenant, par rapport à la composition, le composant (a) en une quantité de 0,01 à 10 % en poids, notamment de 0,1 à 9 % en poids, avantageusement de 0,5 à 8 % en poids, de préférence de 1 à 7 % en poids, de manière particulièrement préférée de 2 à 6 % en poids, de manière tout particulièrement préférée de 3 à 6 % en poids ; et/ou
dans lequel le composant (b) est incorporé dans la composition pharmaceutique, la composition contenant, par rapport à la composition, le composant (b) en une quantité de 0,001 à 2 % en poids, notamment de 0,005 à 1,5 % en poids, avantageusement de 0,01 à 1,2 % en poids, de préférence de 0,02 à 1,0 % en poids, de manière particulièrement préférée de 0,03 à 0,5 % en poids, de manière tout particulièrement préférée de 0,04 à 0,2 % en poids ; et/ou
dans lequel le composant (a) est utilisé et/ou préparé pour l'administration conjointement avec et/ou en combinaison avec le composant (b) sous la forme d'un système aqueux, notamment sous la forme d'un système aqueux monophasé, de préférence sous la forme d'une solution aqueuse ou d'une solubilisation aqueuse, et/ou dans lequel le composant (a) est utilisé et/ou préparé pour l'administration conjointement avec et/ou en combinaison avec le composant (b) avec un excipient ou un véhicule à base d'eau, et/ou dans lequel le composant (a) est utilisé et/ou préparé pour l'administration conjointement avec et/ou en combinaison avec le composant (b) sous la forme d'une solution aqueuse transparente, incolore.

5. Agent thérapeutique combiné selon l'une quelconque des revendications précédentes destiné à une utilisation selon l'une quelconque des revendications précédentes,
dans lequel le composant (a) est utilisé et/ou préparé pour l'administration conjointement avec et/ou en combinaison avec le composant (b) dans la composition pharmaceutique ayant un pH dans la plage allant de 5,0 à 6,2, notamment dans la plage allant de 5,0 à 6,0, avantageusement dans la plage allant de 5,1 à 6,0, de préférence dans la plage allant de 5,2 à 5,9 ;
notamment dans lequel au moins un système tampon chimique, notamment un ou plusieurs sels tampons, est utilisé pour ajuster et/ou maintenir constant le pH de la composition pharmaceutique, un système tampon dihydrogénophosphate/monohydrogénophosphate (« (système) tampon H₂PO₄⁻/HPO₄²⁻ » ou « (système) tampon phosphate »), notamment un système tampon dihydrogénophosphate alcalin/monohydrogénophosphate alcalin, étant de préférence utilisé en tant que système tampon chimique, notamment dans lequel le système tampon dihydrogénophosphate/monohydrogénophosphate est utilisé avec un rapport molaire dihydrogénophosphate/monohydrogénophosphate de plus de 5:1, notamment dans la plage allant de 5:1 à 110:1, de manière particulièrement préférée dans la plage allant de 6:1 à 105:1, de manière tout particulièrement préférée dans la plage allant de 7:1 à 100:1.

6. Agent thérapeutique combiné selon l'une quelconque des revendications précédentes destiné à une utilisation selon l'une quelconque des revendications précédentes,
dans lequel les composants (a) et (b) sont préparés pour l'administration conjointement avec et/ou en combinaison avec (c) au moins un conservateur et/ou un désinfectant, les composant (a), (b) et (c) étant incorporés ou présents dans la composition pharmaceutique ou un médicament ;
notamment dans lequel le conservateur et/ou le désinfectant sont choisis dans le groupe constitué par (i) les chlorures d'alkylbenzyldiméthylammonium, notamment les chlorures d'alkyle en C₈-C₁₈-benzyldiméthylammonium, et les mélanges de différents chlorures d'alkylbenzyldiméthylammonium, de préférence le chlorure de benzalkonium, (ii) les esters de l'acide para-hydroxybenzoïque et les mélanges de différents esters de l'acide para-hydroxybenzoïque, de préférence les esters Nipa, (iii) la chlorhexidine, ainsi que les combinaisons des composés susmentionnés et de manière particulièrement préférée le chlorure de benzalkonium ; et/ou
notamment dans lequel le composant (c), de préférence sous la forme de chlorure de benzalkonium, est administré en une dose individuelle dans la plage allant de 0,001 mg à 5 mg, notamment dans la plage allant de 0,005 mg à 2 mg, de préférence dans la plage allant de 0,01 mg à 1 mg, ou dans lequel le composant (c), de préférence sous la forme de chlorure de benzalkonium, est préparé pour l'administration en une dose individuelle dans la plage allant de 0,005 mg à 2 mg, de préférence dans la plage allant de 0,01 mg à 1 mg, notamment dans lequel 2 à 10 doses individuelles par jour, notamment 3 à 5 doses individuelles par jour, sont administrées, de préférence réparties sur la journée, et/ou notamment dans lequel le composant (c), de préférence sous la forme de chlorure de benzalkonium, est préparé pour l'administration en 2 à 10 doses individuelles par jour, notamment 3 à 5 doses individuelles par jour, de préférence réparties sur la journée ; et/ou
notamment dans lequel le composant (c) est incorporé dans la composition pharmaceutique, la composition contenant, par rapport à la composition, le composant (c) en une quantité de 0,001 à 10 % en poids, notamment de 0,005 à 5 % en poids, avantageusement de 0,01 à 2 % en poids, de préférence de 0,01 à 1 % en poids, de manière particulièrement préférée de 0,01 à 0,5 % en poids.

7. Agent thérapeutique combiné selon l'une quelconque des revendications précédentes destiné à une utilisation selon l'une quelconque des revendications précédentes,
dans lequel les composants (a) et (b) et éventuellement (c) sont préparés pour l'administration conjointement avec et/ou en combinaison avec (d) au moins un glycosaminoglycane de préférence acide ou ses sels ou dérivés physiologiquement inoffensifs, notamment l'acide hyaluronique ou ses sels physiologiquement inoffensifs, les composants (a), (b), (c) et (d) étant incorporés ou présents dans la composition pharmaceutique ;
notamment dans lequel le composant (d) est incorporé dans la composition pharmaceutique, la composition contenant, par rapport à la composition, le composant (d) en une quantité de 0,0001 à 10 % en poids, notamment de 0,001 à 5 % en poids, de préférence de 0,01 à 2 % en poids.

8. Agent thérapeutique combiné selon l'une quelconque des revendications précédentes destiné à une utilisation selon l'une quelconque des revendications précédentes,
dans lequel les composants (a) et (b) et éventuellement (c) et/ou (d) sont préparés pour l'administration conjointement avec et/ou en combinaison avec (e) de l'ectoïne ou au moins un dérivé d'ectoïne, notamment une hydroxyectoïne, les composants (a), (b), (c), (d) et (e) étant incorporés ou présents dans la composition pharmaceutique ;
notamment dans lequel le composant (e) est incorporé dans la composition pharmaceutique, la composition contenant, par rapport à la composition, le composant (e) en une quantité de 0,0001 à 10 % en poids, notamment de 0,001 à 5 % en poids, de préférence de 0,01 à 2 % en poids.

9. Agent thérapeutique combiné selon l'une quelconque des revendications précédentes destiné à une utilisation selon l'une quelconque des revendications précédentes,
dans lequel les composants (a) et (b) et éventuellement (c), (d) et/ou (e) sont préparés pour l'administration conjointement avec et/ou en combinaison avec (f) du chlorure de sodium, les composants (a), (b), (c), (d), (e) et (f) étant incorporés ou présents dans la composition pharmaceutique ;
notamment dans lequel le composant (f) est incorporé dans la composition pharmaceutique, la composition contenant, par rapport à la composition, le composant (e) en une quantité de 0,001 à 5 % en poids, notamment de 0,01 à 2 % en poids, de préférence de 0,1 à 1 % en poids.

10. Agent thérapeutique combiné selon l'une quelconque des revendications précédentes destiné à une utilisation selon l'une quelconque des revendications précédentes, dans lequel tous les agents actifs et/ou ingrédients, notamment les composants (a) et (b) et éventuellement (c), (d), (e) et/ou (f), sont incorporés dans la composition pharmaceutique, la composition présentant une osmolalité dans la plage allant de 300 à 600 mosm/kg, notamment dans la plage allant de 310 à 550 mosm/kg, avantageusement dans la plage allant de 300 à 525 mosm/kg, de préférence dans la plage allant de 325 à 510 mosm/kg, de manière particulièrement préférée dans la plage allant de 350 à 500 mosm/kg.

11. Agent thérapeutique combiné selon l'une quelconque des revendications précédentes destiné à une utilisation selon l'une quelconque des revendications précédentes,
dans lequel tous les agents actifs et/ou ingrédients, notamment les composants (a) et (b) et éventuellement (c), (d), (e) et/ou (f), sont incorporés dans la composition pharmaceutique, la composition présentant à une température de 20 °C et à une pression de 1 013,25 mbar une densité relative, par rapport à l'eau pure, dans la plage allant de 1,001 à 1,2, notamment dans la plage allant de 1,005 à 1,15, de préférence dans la plage allant de 1,005 à 1,105 ; et/ou
dans lequel tous les agents actifs et/ou ingrédients, notamment les composants (a) et (b) et éventuellement (c), (d), (e) et/ou (f), sont incorporés dans la composition pharmaceutique, la composition étant stable à des températures dans la plage allant de 20 °C à 50 °C, à une pression de 1 013,25 mbar et à une humidité relative de l'air dans la plage allant de 50 % à 90 % pendant au moins 6 mois, notamment au moins 12 mois, avantageusement au moins 24 mois, de préférence au moins 36 mois, notamment stable au stockage.

12. Agent thérapeutique combiné selon l'une quelconque des revendications précédentes destiné à une utilisation selon l'une quelconque des revendications précédentes,
dans lequel tous les agents actifs et/ou ingrédients, notamment les composants (a) et (b) et éventuellement (c), (d), (e) et/ou (f), sont incorporés dans la composition pharmaceutique, la composition présentant une teneur en produit(s) de décomposition du composant (b), notamment en impureté A, d'au plus 5 % en poids, notamment d'au plus 3 % en poids, de préférence d'au plus 2 % en poids, de manière particulièrement préférée d'au plus 1 % en poids, par rapport à l'agent actif (b), notamment également après stockage du médicament à des températures dans la plage allant de 20 °C à 50 °C, à une pression de 1 013,25 mbar et à une humidité relative de l'air dans la plage allant de 50 % à 90 % pendant au moins 6 mois, notamment au moins 12 mois, avantageusement au moins 24 mois, de préférence au moins 36 mois, et/ou
dans lequel dans lequel tous les agents actifs et/ou ingrédients, notamment les composants (a) et (b) et éventuellement (c), (d), (e) et/ou (f), sont incorporés dans la composition pharmaceutique, la composition présentant une teneur en produit(s) de décomposition du composant (a), notamment un aminopropanol et/ou D-pantolactone, à chaque fois d'au plus 5 % en poids, notamment à chaque fois d'au plus 3 % en poids, de préférence à chaque fois d'au plus 2 % en poids, de manière particulièrement préférée à chaque fois d'au plus 1 % en poids, par rapport à l'agent actif (a), notamment également après stockage du médicament à des températures dans la plage allant de 20 °C à 50 °C, à une pression de 1 013,25 mbar et à une humidité relative de l'air dans la plage allant de 50 % à 90 % pendant au moins 6 mois, notamment au moins 12 mois, avantageusement au moins 24 mois, de préférence au moins 36 mois.

13. Agent thérapeutique combiné selon l'une quelconque des revendications précédentes
destiné à une utilisation pour le traitement de rhinites de tout type, notamment *Rhinitis acuta, Rhinitis attergica, Rhinitis atrophicans, Rhinitis hyperplastica* ou *hypertrophicans, Rhinitis mutilans, Rhinitis nervosa* ou *vasomotorica* ou *Rhinitis pseudomembranacea,* de préférence *Rhinitis*
*acuta ;* et/ou
destiné à une utilisation pour le traitement topique prophylactique et/ou curatif de rhinites, notamment *Rhinitis acuta.*

14. Agent thérapeutique combiné selon l'une quelconque des revendications précédentes destiné à une utilisation selon l'une quelconque des revendications précédentes,
dans lequel tous les agents actifs et/ou ingrédients, notamment les composants (a) et (b) et éventuellement (c), (d), (e) et/ou (f), sont incorporés dans la composition pharmaceutique, la composition étant administrée et/ou préparée pour l'administration au moyen d'un dispositif d'application, de préférence sous la forme d'un contenant muni d'un dispositif de formation de gouttes ou de pulvérisation ; notamment dans lequel le dispositif d'application comprend un dispositif de pulvérisation pour l'application uniforme de la composition en une quantité par pulvérisation dans la plage allant de 25 µl à 300 µl, notamment dans la plage allant de 50 µl à 200 µl, de préférence dans la plage allant de 75 µl à 125 µl ; et/ou
dans lequel le dispositif d'application comprend un contenant de stockage ayant un volume dans la plage allant de 5 ml à 100 ml, notamment de 10 ml à 50 ml.

15. Pantothénol (dexpanthénol) ou ses esters physiologiquement inoffensifs et/ou acide pantothénique ou ses sels physiologiquement inoffensifs, destinés à une utilisation pour le traitement prophylactique et/ou curatif de rhinites par application nasale dans le but de réduire la résorption systémique d'un sympathomimétique alpha à base d'imidazoline, le pantothénol (dexpanthénol) ou ses esters physiologiquement inoffensifs et/ou l'acide pantothénique ou ses sels physiologiquement inoffensifs étant administrés conjointement avec et/ou en combinaison avec le sympathomimétique alpha à base d'imidazoline ou ses sels physiologiquement inoffensifs.
